# EUROPÄISCHE PATENTSCHRIFT

(11) **EP 2 617 867 B1**
(45) Veröffentlichungstag und Bekanntmachung des Hinweises auf die Patenterteilung: **09.03.2022**
(21) Anmeldenummer: 13000319.7
(22) Anmeldetag: 22.01.2013
(51) Int. Cl.: C11D 3/37, C11D 7/26, C23C 22/50, C23C 22/74, C23F 11/12, C23G 1/08, C09D 5/08, C11D 11/00, A61L 2/24, A61L 2/18

(54) **Verfahren und Zusammensetzung zur Aufbereitung medizinischer Instrumente**
Method and composition for preparing medical instruments
Procédé et composition de préparation des instruments médicaux

(30) Priorität: 23.01.2012 EP 12000395
(43) Veröffentlichungstag der Anmeldung: 24.07.2013
(73) Patentinhaber: Schülke & Mayr GmbH, 22851 Norderstedt (DE)
(72) Erfinder: Cuffaro, Concetta, D-60327 Frankfurt am Main (DE); Lindner, Dirk, D-63303 Dreieich-Offenthal (DE)
(74) Vertreter: LKGLOBAL Lorenz & Kopf PartG mbB Patentanwälte

(56) Entgegenhaltungen:
- EP-A1- 0 666 306
- EP-A1- 1 146 144
- WO-A1-01/30403
- WO-A1-2004/074372
- WO-A1-2007/119059
- WO-A1-2008/107082
- GB-A- 2 097 024
- US-A- 4 381 950
- US-A- 5 330 588
- US-A- 5 534 177
- US-A1- 2006 030 505
- US-A1- 2006 270 571
- US-B2- 6 762 160
- LEE V KREMER: "Improvements in passivation using citric acid formulations", MEDICAL DEVICE MATERIALS, PROCEEDINGS FROM THE MATERIALS & PROCESSES FOR MEDICAL DEVICES CONFERENCE, ANAHEIM, CA, UNITED STATES, SEPT. 8-10, 2003, ASM INTERNATIONAL, USA, 1 January 2003 (2003-01-01), pages 87-90, XP009101025, ISBN: 978-0-87170-798-7

## Beschreibung

### GEBIET DER ERFINDUNG

Die vorliegende Erfindung betrifft ein Verfahren zur Verstärkung der Passivschicht auf der Oberfläche des Chrom-haltigen Stahls, sowie ein Verfahren zur Aufbereitung der Oberfläche eines Chrom-haltigen Stahls, wobei der Stahl vorzugsweise Teil eines medizinischen Instruments ist oder als medizinisches Instrument ausgebildet ist. Des weiteren wird eine wässerige Lösung, welche zur Verstärkung der Passivschicht verwendet werden kann, eine Vorrichtung zur Durchführung der Verfahren aufweisend die wässerige Lösung, ein medizinisches Instrument, welches den behandelten Chrom-haltigen Stahl aufweist, ein derartiges medizinisches Instrument zur Verwendung bei der therapeutischen Behandlung, sowie Verwendungen der erfindungsgemäßen Verfahren und der wässrigen Lösung beschrieben.

### HINTERGRUND DER ERFINDUNG

Im Krankenhausalltag sind Stahl-haltige Medizinprodukte, insbesondere medizinische Instrumente, aber auch medizinische Vorrichtungen wie Reinigungs- und Desinfektionsgeräte (RDG) oder Sterilisatoren, regelmäßig oberflächenschädigenden Einflüssen ausgesetzt, die ihre Lebensdauer vermindern. Zudem erhöhen fehlerhafte Oberflächen das Infektionsrisiko, z.B. durch stärkeres Anhaften von Verunreinigungen, können aber auch die generelle Fehlfunktion eines medizinischen Instruments bedingen. Sie stellen somit ein generelles Gesundheitsrisiko für Personal und Patient dar. Typische oberflächenschädigende Einflüsse sind zunächst die im medizinischen Bereich übliche starke mechanische Beanspruchung sowie korrosiv wirkende Bedingungen. Untere Letztere fallen der im Klinikalltag übliche und unvermeidbare Kontakt mit Blut und verschiedenen wässrigen Lösungen, insbesondere Kochsalzlösungen, in denen die Instrumente z.B. direkt nach dem Gebrauch / der Anwendung zwischengelagert werden. Übliche Verfahren zur Aufbereitung medizinischer Instrumente und Vorrichtungen beinhalten zunächst das Reinigen, umfassend das Abspülen der Instrumente oder der Vorrichtung mit Wasser und Behandlung mit wässerigen Reinigungszusammensetzungen nach der Anwendung. Nach der Reinigung werden die Instrumente desinfiziert und im Anschluss in der Regel sterilisiert. Die Sterilisation erfolgt oft durch heißen Wasserdampf unter Druck im Autoklaven. Es hat sich gezeigt, dass selbst der regelmäßige Kontakt mit Reinstwasser bei hohen Temperaturen im Rahmen der Desinfektion bzw. mit dem bei Sterilisationsprozessen häufig verwendeten Wasserdampf, sich schädigend auf die Stahl-haltigen Oberflächen auswirken kann.

Auf legierten Chrom-haltigen Stahloberflächen bildet sich üblicherweise während oder nach seiner Herstellung eine Passivschicht aus oder kann nach seiner Herstellung erzeugt werden, welche die Korrosion der Metalloberfläche verhindern oder zumindest erniedrigen kann. Diese Passivschichten bestehen gewöhnlich aus Chromoxid, geringeren Mengen an Eisenoxid und den Oxiden weiterer evtl. zulegierter Metalle. Solche Passivschichten weisen üblicherweise eine Schichtdicke im Bereich von 1,0 bis 3,0 nm auf. Das Verhältnis von oxidisch gebundenem Chrom zu oxidisch gebundenem Eisen kann beispielsweise durch die bekannte Auger-Elektronenspektroskopie und / oder durch ESCA-Analyse in Kombination mit bekannten Sputtertechniken ermittelt werden.

Verfahren zur Reinigung von Stahloberflächen und / oder Erhöhung der Korrosionsbeständigkeit von Stahloberflächen sind bekannt.

EP 1 454 637 A1 betrifft ein Verfahren zur Reinigung und Sterilisation medizinischer Instrumente, wobei während der Reinigung eine Reinigungszusammensetzung in Kombination mit einer Peroxidverbindung verwendet wird.

WO 2008/107082 A1 betrifft ein Verfahren zur Verbesserung der Temperatur- und Korrosionsbeständigkeit von Edelstahl, wobei ein komplexierend wirkendes Agens in Kombination mit einem Oxidationsmittel eingesetzt wird. Anschließend wird der Edelstahl erhöhter Temperatur in einer Sauerstoff-haltigen Atmosphäre ausgesetzt.

EP 0 595 686 B1 betrifft ein Beizverfahren für Stahlwerkstoffe, vorzugsweise Bandstahl, wobei wässerige Lösungen organischer Säuren, beispielsweise Maleinsäureanhydrid in einer Menge von mindestens 5 Gew.-% bezogen auf die wässerige Lösung, in Kombination mit Gluconsäure und Zitronensäure eingesetzt werden.

WO 2006/134116 A1 betrifft ein Verfahren zur Passivierung von Metalloberflächen mit Polymeren, welche Säuregruppen aufweisen. Dabei wird vorzugsweise Bandstahl mit den Polymeren beschichtet (coil coating).

DE 10 2004 041142 A1 betrifft ein Verfahren zum Passivieren von metallischen Oberflächen unter Verwendung von Homo- oder Copolymeren von Itaconsäure. Dabei wird vorzugsweise Bandstahl mit den Polymeren beschichtet (coil coating).

Es sind auch handelsübliche Zusammensetzungen bekannt, die zur Erhöhung der Korrosionsbeständigkeit von Stahloberflächen, beispielsweise Stahloberflächen von medizinischen Instrumenten, verwendet werden können. Derartige Zusammensetzungen enthalten Salpetersäure oder Zitronensäure.

Die WO 01/30403 A1 beschreibt ein modifiziertes Medizinprodukt zur Abgabe eines pharmazeutisch aktiven Materials und betrifft verschiedene Modifikationen der metallischen oder polymeren Komponente in dem modifizierten Medizinprodukt.

Die EP 1146144 A1 beschreibt eine oberflächenbehandelnde Zusammensetzung für Metall, die Al, Mg und Mn-Metallionen, ein wasserlösliches organisches Harz, eine Säure und Wasser umfasst. Die Zusammensetzung ist chromfrei.

L. V. Kremer et al. beschreibt in "Improvements in passivation using citric acid formulations" (Medical Device Materials, Proceedings from the Materials & Processing for Medical Devices Conference, Anaheim, CA, US, September 8-10, 2003, Seiten 87-90), dass die Bildung von unerwünschten NO_{(X)}-Gasen aus Salpetersäure durch die Verwendung von Lösungen, die auf Zitronensäure basieren, vermieden werden kann.

EP 1 146 144 A1 offenbart die Verwendung einer Säure-haltigen Lösung, welche 10 Gew.% wasserlösliches Copolymerisat von Maleinsäure mit Acrylsäure enthält, zur Behandlung von Edelstahl (SUS 304), um eine Konversionsbeschichtung mit verbesserter Korrosionsbeständigkeit und Leitfähigkeit bereitzustellen.

### AUFGABE DER ERFINDUNG

Eine Aufgabe der Erfindung ist es, ein Verfahren bereit zu stellen, mit welchem die Korrosion einer Chrom-haltigen Stahloberfläche vermindert werden kann, wobei dieses Verfahren vorzugsweise im Krankenhausbetrieb leicht einsetzbar, möglichst standardisierbar und / oder automatisierbar sein soll.

### ZUSAMMENFASSUNG DER ERFINDUNG

Diese Aufgabe wird mit einem Verfahren zur Aufbereitung der Oberfläche eines Chrom-haltigen Stahls, insbesondere zur Verstärkung der Passivschicht auf der Oberfläche eines Chrom-haltigen Stahls, gelöst, vorzugsweise eines Stahls, welcher für medizinische Instrumente oder medizinische Vorrichtungen verwendet wird. Dabei wird das Verfahren so durchgeführt, dass eine auf der Oberfläche des Stahls vorhandene Passivschicht verstärkt wird. Diese Verstärkung kann durch Erhöhen des Chrom / Eisen-Verhältnisses in der Passivschicht oder durch Erhöhen der Schichtdicke der Passivschicht erfolgen oder durch Erhöhen des Chrom / Eisen-Verhältnisses in der Passivschicht und durch Erhöhen der Schichtdicke der Passivschicht.

Gemäß eines ersten Aspekts betrifft die Erfindung ein Verfahren zur Verstärkung der Passivschicht auf der Oberfläche eines Chrom-haltigen Stahls durch Erhöhen des Chrom / Eisen-Verhältnisses in der Passivschicht und / oder durch Erhöhen der Schichtdicke der Passivschicht, dadurch gekennzeichnet, dass es mindestens die Stufe (ii) aufweist und optional vor oder gleichzeitig mit Stufe (ii) die Stufe (i) ausgeführt wird:
(i) Reinigen der Oberfläche;
(ii) Kontaktieren der Oberfläche des Stahls mit einer Säure-haltigen wässerigen Lösung, wobei die Säure ausgewählt ist aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure, wobei das wasserlösliches Copolymerisat von Maleinsäure oder Maleinsäureanhydrid oder Itaconsäure ein Copolymerisat von Maleinsäure oder Maleinsäureanhydrid oder Itaconsäure mit ethylenisch ungesättigten Verbindungen oder mit anderen ungesättigten Verbindungen ist;

wobei der Gewichtsanteil von Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure in der Säure-haltigen wässerigen Lösung kleiner als 5 Gew.-% ist bezogen auf das Gesamtgewicht der Lösung ist,
wobei das Kontaktieren der Oberfläche mit der Säure-haltigen wässrigen Lösung so durchgeführt wird, dass das Chrom / Eisen-Verhältnis in der Passivschicht auf einen Wert ≥ 3 und die Schichtdicke der Passivschicht auf einen Wert > 4 nm erhöht wird.

In eine Ausführungsform weist die Säure-haltige wässerige Lösung zusätzlich eine oder mehrere der folgenden Verbindungen oder Zusammensetzungen auf: Ameisen-, Essig-, Malon-, Bernstein-, Wein-, Apfel-, Zitronen-, Glutar-, Glucon-, Milch-, Benzoe-, Ethylendiamintetraessig-, Nitrilotriessig-, Salpetersäure; Oxidationsmittel; Reinigungszusammensetzung.

In einer weiteren Ausführungsform liegt die Säure-haltige Lösung als Flüssigkeit vor; oder als Schaum; oder als Gel; oder als Paste; oder als Spray; und / oder in einem Vlies; in einem Ultraschallbad; in einer Spülmaschine oder in einem Reinigungs- und Desinfektionsgerät.

In einer Ausführungsform betrifft die Erfindung ein Verfahren zur Aufbereitung der Oberfläche eines Chrom-haltigen Stahls, dadurch gekennzeichnet, dass es mindestens die folgenden Stufen (i) bis (iii) aufweist, welche nacheinander ausgeführt werden:
(i) Reinigen der Oberfläche;
(ii) Kontaktieren der Oberfläche mit einer Säure-haltigen wässerigen Lösung und / oder einem Oxidationsmittel;
(iii) Desinfizieren der Oberfläche.

In einer Ausführungsform weist Stufe (i) die Stufen (i') und (i‴) und optional (i") auf; und Stufe (iii) weist die Stufen (iii') bis (iii‴) auf:
(i') Abspülen der Oberfläche mit Wasser; und vorzugsweise nach dem Abspülen, Entfernen des Wassers von der Oberfläche;
(i") optional nach Stufe (i'), Wiederholung der Stufe (i');
(i‴) nach oder während Stufe (i') oder (i"), Kontaktieren der Oberfläche mit einer Reinigungszusammensetzung; und nach dem Kontaktieren, Entfernen der Reinigungszusammensetzung von der Oberfläche;
(iii') nach Stufe (ii), Abspülen der Oberfläche mit Wasser; und vorzugsweise nach dem Abspülen, Entfernen des Wassers von der Oberfläche
(iii") Spülen der Oberfläche mit entsalztem Wasser, wobei das entsalzte Wasser erhitzt wird, um die Oberfläche zu desinfizieren; und vorzugsweise nach dem Erhitzen, Entfernen des entsalzten Wassers von der Oberfläche;
(iii‴) nach Stufe (iii"), Trocknen der Oberfläche.

In einer Ausführungsform weist die Säure-haltige wässerige Lösung eine oder mehrere der folgenden Verbindungen auf: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure; und / oder wobei das Oxidationsmittel ausgewählt ist aus einer Peroxidverbindung, vorzugsweise aus Wasserstoffperoxid oder Peressigsäure; oder wobei die Säure der Säure-haltigen Lösung ausgewählt ist aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure.

In einer Ausführungsform weist die Säure-haltige wässerige Lösung zusätzlich eine oder mehrere der folgenden Verbindungen auf: Ameisen-, Essig-, Malon-, Bernstein-, Wein-, Apfel-, Zitronen-, Glutar-, Glucon-, Milch-, Benzoe-, Ethylendiamintetraessig-, Nitrilotriessig-, Salpetersäure.

In einer Ausführungsform ist der Stahl Teil eines medizinischen Instruments oder ist als medizinisches Instrument ausgebildet.

Beschrieben wird ferner eine wässrige Lösung aufweisend eine oder mehrere der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure, dadurch gekennzeichnet, dass der Gewichtsanteil der Verbindung oder der Verbindungen in der Lösung kleiner als 5 % ist bezogen auf das Gesamtgewicht der Lösung.

Beschrieben wird auch eine Vorrichtung zur Durchführung des Verfahrens, wobei die Vorrichtung vorzugsweise eine Spülmaschine oder ein Reinigungs- und Desinfektionsgerät ist.

In der Vorrichtung kann mittels einer Programmierung mindestens Stufe (ii) aufgerufen und durchgeführt werden.

Beschrieben wird weiter ein medizinisches Instrument, welches eine Oberfläche eines Chrom-haltigen Stahls aufweist, wobei der Stahl Teil des medizinischen Instruments ist oder als das medizinische Instrument ausgebildet ist, dadurch gekennzeichnet, dass es nach einem Verfahren herstellbar ist gemäß des ersten Aspekts oder gemäß des zweiten Aspekts; oder ein medizinisches Instrument, welches eine Oberfläche eines Chrom-haltigen Stahls aufweist, wobei der Stahl Teil des medizinischen Instruments ist oder als das medizinische Instrument ausgebildet ist, und wobei die Oberfläche eine Passivschicht aufweist, dadurch gekennzeichnet, dass in der Passivschicht das Chrom / Eisen-Verhältnis ≥ 1 oder ≥ 1.5 ist gemessen über eine Schichtdicke der Passivschicht von 8 nm, vorzugsweise 12 nm.

Beschrieben wird weiter ein medizinisches Instrument wie vorstehend beschrieben zur Verwendung bei der therapeutischen Behandlung eines Menschen oder eines Tieres.

Beschrieben wird ferner die Verwendung des Verfahrens zur Herabsetzung des Haftungsvermögens von Prionen an einer Chrom-haltigen Stahloberfläche.

Gemäß eines zweiten Aspekts betrifft die Erfindung die Verwendung einer Säure-haltigen wässrigen Lösung zur Verstärkung der Passivschicht auf der Oberfläche eines Chrom-haltigen Stahls durch Erhöhen des Chrom /Eisen Verhältnisses in der Passivschicht und /oder durch Erhöhen der Schichtdicke der Passivschicht, wobei die Säure ausgewählt ist aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure, wobei das wasserlösliches Copolymerisat von Maleinsäure oder Maleinsäureanhydrid oder Itaconsäure ein Copolymerisat von Maleinsäure oder Maleinsäureanhydrid oder Itaconsäure mit ethylenisch ungesättigten Verbindungen oder mit anderen ungesättigten Verbindungen ist, wobei der Gewichtsanteil von Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure in der Säure-haltigen wässrigen Lösung kleiner als 5 Gew.% bezogen auf das Gesamtgewicht der Lösung ist.

### DETAILLIERTE BESCHREIBUNG DER ERFINDUNG

Alle nachfolgenden Definitionen sind Definitionen im Sinne der Erfindung.

### Erster Aspekt der Erfindung

Der ***erste Aspekt*** der Erfindung betrifft ein Verfahren zur Verstärkung der Passivschicht auf der Oberfläche eines Chrom-haltigen Stahls. In einer Ausführungsform wird die Verstärkung der Passivschicht durch Erhöhen des Chrom-Eisen-Verhältnisses in der Passivschicht oder durch Erhöhung der Schichtdicke der Passivschicht erreicht. In einer weiteren Ausführungsform wird die Verstärkung der Passivschicht durch Erhöhen des Chrom-Eisen-Verhältnisses in der Passivschicht und durch Erhöhung der Schichtdicke der Passivschicht erreicht.

Der Begriff *"Oberfläche"* bedeutet die Fläche des Stahls welche erfindungsgemäß behandelt wird, sowie die oberste Schicht oder die oberen Schichten des Stahl. Der Begriff schließt somit eine auf dem Stahl vorhandene Passivschicht ein.

Der Begriff *"Chrom-haltiger Stahl"* bedeutet einen Stahl, der als Komponente, vorzugsweise als Legierungskomponente, Chrom enthält. Der Begriff umfasst somit auch Stähle, die als Chrom-Stahl, Chrom/Nickel-Stahl oder Chrom/Molybdän-Stahl bezeichnet werden können. Titan-Stahl, welcher Chrom enthält, fällt gleichfalls unter diese Bezeichnung.

In einer Ausführungsform ist der Chrom-haltige Stahl ein Stahl, der für medizinische Anwendungen eingesetzt wird. Typische Stähle, die in medizinischen Vorrichtungen, wie RDGs oder Sterilisatoren verwendet werden, sind Stähle mit der Bezeichnung 1.4031 und 1.4404. Stähle für medizinische Instrumente werden zum Beispiel definiert in EN ISO 7153-1. Derartige Stähle sind auch unter den Bezeichnungen 1.4021 und 1. 4024 bekannt.

In einer Ausführungsform wird der Chrom-Stahl somit ausgewählt aus einem Stahl mit der Bezeichnung 1.4031, 1.4404, 1.4021 und / oder 1. 4024.

Wird in der vorliegenden Offenbarung auf das *"Verhältnis von Chrom zu Eisen"* (auch *"Chrom* / *Eisen Verhältnis"*) Bezug genommen, so handelt es sich um das atomare Verhältnis von oxidisch gebundenem Chrom zu oxidisch gebundenem Eisen.

Der Begriff *"Passivschicht"* bedeutet eine Schicht auf der Oberfläche des Stahls, welche die Korrosion der Metalloberfläche verhindert oder zumindest erniedrigt. Im Falle von Chrom-haltigen Stahl weist die Passivschicht Chrom- und Eisenoxide auf. Die Schichtdicke der Passivschicht liegt üblicherweise im Bereich von 1,0 bis 3,0 nm, kann sich jedoch auch weiter in das Innere des Stahls erstrecken. In Chrom-haltigen Stahl, welcher noch nicht dem erfindungsgemäßen Verfahren unterzogen wurde, ist das Chrom / Eisen-Verhältnis in der Oberfläche gewöhnlich < 1. Dies kann beispielsweise durch die bekannte Auger-Elektronenspektroskopie und / oder durch ESCA-Analyse in Kombination mit bekannten Sputtertechniken ermittelt werden.

Das Verfahren gemäß des ***ersten Aspekts*** ist dadurch gekennzeichnet, dass es mindestens die Stufe (ii) und optional vor oder gleichzeitig mit Stufe (ii) die Stufe (i) aufweist:
(i) Reinigen der Oberfläche;
(ii) Kontaktieren der Oberfläche des Stahls mit einer Säure-haltigen wässerigen Lösung, wobei die Säure ausgewählt ist aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure.

### Stufe (i)

Der Begriff *"Reinigung"* bedeutet einen Vorgang, bei dem eine Zusammensetzung auf die Oberfläche des Chrom-haltigen Stahls einwirkt, derart, dass Rückstände von der Oberfläche abgelöst und entfernt werden ohne dass bestimmungsgemäß eine Abtötung / Inaktivierung von Mikroorganismen stattfindet bzw. beabsichtigt ist. Typische Rückstände sind beispielsweise Blut, Serum, Lymphe, Proteine, Geweberückstände, Fette, Kohlenhydrate und andere Körpersubstanzen oder Ausscheidungen eines menschlichen oder tierischen Körpers, d.h. in der Regel Rückstände, welche ein Peptid oder ein Protein aufweisen, aber auch Staub bzw. chemische Substanzen.

Die zum Reinigen der Oberfläche verwendete Zusammensetzung, die Reinigungszusammensetzung, kann oberflächenaktive Verbindungen aufweisen, wie sie gewöhnlich für Reinigungszwecke in Spül- und Waschmitteln verwendet werden. Die Reinigungszusammensetzung kann alkalisch sein und / oder gegebenenfalls Alkohole enthalten. In einer weiteren Ausführungsform kann zum Reinigen eine Enzym-enthaltende Reinigungszusammensetzung verwendet werden. Derartige Zusammensetzungen sind besonders zum Entfernen von Peptiden oder Proteinen von der Stahloberfläche geeignet.

Die in der Reinigungszusammensetzung enthaltenen Enzyme können sein: ein oder mehrere Enzyme ausgewählt aus der Gruppe bestehend aus Amylase, Lipase, Gluconase, Cellulase, Peroxidase, Protease oder eine Mischung davon. Geeignete Quellen für derartige Enzyme sind Pflanzen, Tiere, Bakterien, Pilze oder Hefen.

In einer Ausführungsform wird eine Enzym-Lösung eingesetzt, welche alkalisch ist, d.h. einen pH-Wert von 8 - 12 aufweist.

Demzufolge weist in einer Ausführungsform Stufe (i) auf:
(i) Reinigen der Oberfläche mit Hilfe eines Enzyms, vorzugsweise bei einem pH-Wert von 8 - 12.

Geeignete weitere Verbindungen, die in einer Reinigungszusammensetzung enthalten sein können, schließen Wasser, Konservierungsstoffe, oberflächenaktive Verbindungen und / oder organische Lösungsmittel ein.

Der Begriff "*Wasser*" schließt Leitungswasser, vollentsalztes oder teilentsalztes Wasser ein.

Der Begriff *"Konservierungsmittel"* bedeutet eine natürliche oder synthetische Substanz, welche der Reinigungszusammensetzung, die im erfindungsgemäßen Verfahren eingesetzt wird, hinzugefügt wird. Dadurch kann die Zersetzung der Reinigungsflüssigkeit durch mikrobielles Wachstum oder durch unerwünschte chemische Veränderungen verhindert oder verringert werden. Einsetzbare Konservierungsmittel sind beispielsweise Calciumpropionat, Natriumnitrat, Sulfite wie beispielsweise Natriumsulfit, Natriumbisulfit, Kaliumhydrogensulfit, sowie das Dinatriumsalz von EDTA, oder Ethanol, Chlorisothiazol oder Methylchlorisothiazol.

Der Begriff *"oberflächenaktive Verbindung"* schließt jede Verbindung ein, die die Oberflächenspannung einer Flüssigkeit oder die die Grenzflächenspannung zwischen zwei Flüssigkeiten oder die die Grenzflächenspannung zwischen einer Flüssigkeit und einem Feststoff herabsetzt. Oberflächenaktive Moleküle sind amphiphil, was bedeutet, dass sie sowohl hydrophobe wie auch hydrophile Gruppen enthalten. Die oberflächenaktiven Verbindungen können anionisch, kationisch, amphoter oder nichtionisch sein.

Der Begriff *"anionische oberflächenaktive Verbindung"* bedeutet oberflächenaktive Verbindungen, welche auf Anionen basieren. In einer Ausführungsform können eingesetzt werden: Sulfate wie beispielsweise Alkylsulfate wie beispielsweise Ammoniumlaurylsulfat oder Natriumlaurylsulfat; Alkylethersulfate wie beispielsweise Natriumlaurethsulfat (Laurylethersulfat) oder Natriummyrethsulfat; Sulfonate wie beispielsweise Dioctylnatriumsulfosuccinat; Fluor-haltige Verbindung wie beispielsweise Perfluoroctansulfonat oder Perfluorbutansulfonat; Alkylbenzolsulfonate; Phosphate wie beispielsweise Alkylaryletherphosphate und Alkyletherposphate; Carboxylate wie beispielsweise Alkylcarboxylate wie beispielsweise Fettsäuresalze wie beispielsweise Natriumstearat; Natriumlaurylsarcosinat; fluorierte Carboxylate wie beispielsweise Perfluoroctanoat und Perfluornonanoat; oder zwei oder mehr davon.

In einer Ausführungsform weist die anionische oberflächenaktive Verbindung ein Salz eines Sulfatesters eines (C1-C10)-Alkohols auf, ein Sulfatestersalz eines alkoxylierten Produkts eines (C8-C20)-Alkohols, ein Alkylbenzolsulfonatsalz, ein Paraffinsulfonatsalz, ein α-Olefinsulfonatsalz, ein α-Sulfofettsäuresalz, ein α-Sulfofettsäurealkyestersalz, oder ein Fettsäuresalz, oder zwei oder mehr davon. In einer Ausführungsform ist die anionische oberflächenaktive Verbindung ein lineares (C10-C14)-Alkylbenzolsulfonsäuresalz wie beispielsweise ein (C12-C14)-Alkybenzolsulfonsäuresalz. Das Gegenion ist vorzugsweise ein Alkalimetall, beispielsweise Natrium und/oder Kalium in Form von Kationen, oder ein Amin wie Monoethanolamin oder Diethanolamin in Form von Ammoniumkationen.

Der Begriff *"kationische oberflächenaktive Verbindung"* bedeutet oberflächenaktive Verbindungen, welche auf Kationen basieren. Der Begriff schließt eine quartäre Ammoniumverbindung ein, wobei der Stickstoff typischerweise Reste trägt ausgewählt aus: Stearyl, Palmityl, Methyl, Benzyl, Butyl. Der Stickstoff kann auch Bestandteil eines gesättigten oder ungesättigten Ringsystem sein, vorzugsweise eines Pyridinrings. Geeignete kationische Verbindungen, welche im Sinne der Erfindung eingesetzt werden können, sind beispielsweise Benzalkoniumhalogenide. Diese können auch eine biozide Wirkung aufweisen.

Der Begriff *"amphotere oberflächenaktive Verbindung"* schließt Verbindungen ein, welche sowohl eine negativ als auch eine positiv geladene funktionelle Gruppe aufweisen, also Zwitterionen. Sie leiten sich von Betain ab. Derartige Verbindungen weisen zumeist eine Carboxylat-Gruppe und eine quartäre Ammoniumgruppe auf. Eine geeignete Verbindung, welche im Sinne der Erfindung eingesetzt werden kann, ist das bekannte Kokosfettsäureamidopropylbetain.

Der Begriff *"nichtionische oberflächenaktive Verbindung"* schließt ein: Fettalkohole wie beispielsweise Cetylalkohol, Stearylalkohol, Cetostearylalkohol, Oleylalkohol; Polyoxyethylenglycolalkylether wie Octaethylenglycolmonododecylether oder Pentaethylenglycolmonododecylether; Polyoxypropylenglycolalkylether; Glucosidalkylether wie Decyl- oder Lauryl- oder Octylglucoside; Polyoxyethylenglycolalkylphenolether wie Polyoxyethylenglycoloctylphenolether; Glycerolalkylether wie Glyceryllaurate; Polyoxyethylenglycolsorbitanalkylester; Sorbitanalkylester; Cocamide; Dodecyldimethylaminoxide; Blockcopolymere von Polyethylenglycol und Polypropylenglycol. In einer weiteren Ausführungsform schließt die nichtionische oberflächenaktive Verbindung ein: einen Polyoxyalkylen(C8-C20)alkylether, ein Alkylpolyglycosid, einen Polyoxyalkylen(C8-C20)alkylphenylether, einen Polyoxyalkylenesorbitanfettsäure(C8-C22)alkylester, einen Polyoxyalkylenglycolfettsäure(C8-C22)alkylester, ein Polyoxyethylen/Polyoxypropylen-Blockcopolymer; oder zwei oder mehr davon. In einer Ausführungsform ist die nichtionische oberflächenaktive Verbindung ein Polyoxyalkylenalkylether.

Der Begriff *"organisches Lösungsmittel"* schließt jedes Lösungsmittel ein, das geeignet ist, Verbindungen zu solubilisieren, welche in den erfindungsgemäßen Verfahren beziehungsweise der erfindungsgemäßen wässerigen Lösung verwendet werden. In einer Ausführungsform ist das organische Lösungsmittel ein Lösungsmittel, welches mit Wasser mischbar ist. In einer Ausführungsform ist das wassermischbare Lösungsmittel ausgewählt aus: (C1-C6)-Alkoholen, (C2-C6)-Diolen, (C2-C4)-Alkylenglycolen, (C3-C24)-Alkylenglycolethern, und Mischungen aus zwei oder mehr davon. In einer weiteren Ausführungsform ist das organische Lösungsmittel Dipropylenglycol. In einer weiteren Ausführungsform ist das organische Lösungsmittel Monoethylenglycol und / oder Propylenglycol, oder eine Mischung davon.

In einer Ausführungsform wird zum Reinigen der Oberfläche eine alkalische Reinigungszusammensetzung eingesetzt. Der Begriff *"alkalisch"* bedeutet einen pH-Bereich von circa 8 - 12, von 10 - 12, oder von 11 - 11,5, wobei der pH-Wert sich auf den pH-Wert der mit der zu reinigenden Oberfläche in Kontakt zu bringenden Lösung bezieht. Der pH-Wert kann demnach auch erst durch anwendungsfertige Verdünnung eines Konzentrats oder durch Zugabe von Alkalien zu einer zuvor nichtalkalischen Reinigungszusammensetzung eingestellt werden und demnach auch erst beim automatischen Einspülen in eine Maschine, z.B. einen RDG, erreicht werden.

In einer Ausführungsform beträgt die Temperatur in Stufe (i) 5 bis 90 °C.

In einer Ausführungsform beträgt die Temperatur in Stufe (i) für einen Zeitraum von mindestens 3, oder mindestens 5, oder mindestens 10 Minuten 50 - 60 °C, zum Beispiel 55 °C.

Zum Beispiel kann die Stufe (i) bei einem pH von 10 - 12, unter Einhalten einer Temperatur von 50 - 60 °C, für mindestens 10 Minuten durchgeführt werden. In einer Ausführungsform werden die Bereiche für den pH-Wert, Temperatur und Reinigungszeit kombiniert.

Geeignete Verfahren zum Durchführen der Reinigungsstufe bestehen zum Beispiel in Sprühverfahren oder Tauchverfahren. In einer Ausführungsform wird das Reinigen durchgeführt unter Anwendung von Ultraschall und / oder wird mechanisch unterstützt, beispielsweise durch Bürsten.

Die Reinigungsstufe kann automatisch in einer Spülmaschine oder einem RDG durchgeführt werden.

### Stufe (ii)

Erfindungsgemäß erfolgt in Stufe (ii) das Kontaktieren der Oberfläche mit einer Säure-haltigen wässerigen Lösung, wobei die Säure ausgewählt ist aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure.

Der Begriff *"Kontaktieren der Oberfläche mit einer Säure-haltigen wässrigen Lösung..."* gemäß Stufe (ii) umfasst ein Verfahren, in welchem sowohl ein Teil der Oberfläche des Chrom-haltigen Stahls wie auch die vollständige Oberfläche des Chrom-haltigen Stahls mit der Lösung bedeckt werden kann. Geeignete Verfahren zum Kontaktieren bestehen zum Beispiel in Sprühverfahren oder Tauchverfahren. In einer Ausführungsform wird das Kontaktieren durchgeführt unter Anwendung von Ultraschall und / oder wird mechanisch unterstützt, beispielsweise durch Bürsten.

Der Begriff *"Maleinsäure* ..." umfasst auch die Salze der Maleinsäure bzw. der weiteren aufgelisteten Verbindungen.

In einer Ausführungsform sind Halbester Ester mit 1-6 Kohlenstoffatomen in der Estergruppe. In einer weiteren Ausführungsform sind die zur Veresterung verwendeten Alkohole lineare oder verzweigte (C1-C6)-Alkohole.

Der Begriff *"wasserlösliches Copolymerisat von Maleinsäure oder Maleinsäureanhydrid oder Itaconsäure"* bedeutet ein Copolymerisat von Maleinsäure oder Maleinsäureanhydrid oder Itaconsäure mit ethylenisch ungesättigten Verbindungen, vorzugsweise Styrol, Acrylsäure oder Acrylsäureester, Methacrylsäure oder Methacrylsäureester oder mit anderen ungesättigten Verbindungen wie zum Beispiel Ethylen, Propylen und / oder Isobutylen. Derartige Copolymerisate sind bekannt oder können nach bekannten Verfahren hergestellt werden.

Der Begriff *"wasserlöslich"* bedeutet, dass sich mindestens ein Gramm des Copolymerisats in 100 ml Wasser auflösen lässt, wobei die Lösung bei 23 °C stabil ist.

Erfindungsgemäß kann die Säure-haltige wässerige Lösung zusätzlich eine oder mehrere der folgenden Verbindungen oder Zusammensetzungen aufweisen: Ameisen-, Essig-, Malon-, Bernstein-, Wein-, Apfel-, Zitronen-, Glutar-, Glucon-, Milch-, Benzoe-, Ethylendiamintetraessig-, Nitrilotriessig-, Salpetersäure; Oxidationsmittel; Reinigungszusammensetzung.

In einer Ausführungsform ist das Oxidationsmittel ausgewählt aus einer Peroxidverbindung, vorzugsweise aus Wasserstoffperoxid oder einer Persäure wie Peressigsäure. In einer Ausführungsform liegt das Oxidationsmittel in einem Lösungsmittel vor, vorzugsweise in Wasser oder in einem organischen Lösungsmittel wie oben definiert.

In einer weiteren Ausführungsform kann auch die Säure-haltige wässerige Lösung mit einem Oxidationsmittel kombiniert werden.

In einer Ausführungsform weist die Säure-haltige wässerige Lösung zusätzlich Salpetersäure auf.

Die Säure-haltige Lösung kann in verschiedenen Aggregatszuständen vorliegen. In einer Ausführungsform kann sie als Flüssigkeit vorliegen; oder als Schaum; oder als Gel; oder als Paste; oder als Spray.

In einer weiteren Ausführungsform kann ein Vlies, d.h. ein textiles Gewebe, beispielsweise mit der Flüssigkeit getränkt werden oder der Schaum oder das Gel oder die Paste auf das Gewebe aufgebracht werden. Dieses Gewebe kann dann dazu verwendet werden, die Säure-haltige wässerige Lösung auf die Chrom-haltige Stahloberfläche aufzubringen, beispielsweise durch Abwischen der Stahloberfläche.

In einer Ausführungsform ist es möglich, dass die Stufen (i) und (ii) in einem Arbeitsgang durchgeführt werden. Dann erfolgen Reinigung und Verstärkung der Passivschicht gleichzeitig oder annähernd gleichzeitig.

In einer weiteren Ausführungsform ist es möglich, dass die Stufen (i) und (ii) getrennt voneinander durchgeführt werden. Dann erfolgen Reinigung und Verstärkung der Passivschicht nacheinander.

Der Begriff "Verstärkung der Passivschicht"bedeutet in einer Ausführungsform das Erhöhen des Chrom / Eisen-Verhältnisses in der Passivschicht und / oder das Erhöhen der Schichtdicke der Passivschicht, wobei hier auf die der Dicke der Schicht Bezug genommen wird, innerhalb derer ein Chrom / Eisen-Verhältnis von mindestens ≥1 vorliegt. Sowohl das Chrom / Eisenverhältnis, also auch die Dicke der Passivschicht, sind mittels ESCA-Tiefenprofilmessungen bestimmbar.

Das Kontaktieren wird so durchgeführt, dass das Chrom / Eisen-Verhältnis in der Passivschicht auf einen Wert ≥ 3 erhöht wird. Eine derartige Passivschicht hat sich als wirksam gegen Korrosionsbildung erwiesen.

Das Kontaktieren wird so durchgeführt, dass die Schichtdicke der Passivschicht auf einen Wert > 4 nm erhöht wird. Eine derartige Passivschicht hat sich als wirksam gegen Korrosionsbildung erwiesen.

Das Kontaktieren wird so durchgeführt, dass das Chrom / Eisen-Verhältnis in der Passivschicht auf einen Wert ≥ 3, und die Schichtdicke der Passivschicht auf einen Wert > 4 nm erhöht wird. Eine derartige Passivschicht hat sich als besonders wirksam gegen Korrosionsbildung erwiesen.

In einer Ausführungsform wird das Kontaktieren so durchgeführt, dass das Chrom / Eisen-Verhältnis in der Passivschicht auf einen Wert ≥ 1,0 oder ≥ 1,5 erhöht wird gemessen über eine Schichtdicke von 8 nm oder mindestens 8 nm, vorzugsweise 12 nm. Eine derartige Passivschicht hat sich als besonders wirksam gegen Korrosionsbildung erwiesen.

In einer Ausführungsform wird die Verstärkung der Passivschicht durch entsprechend langes Kontaktieren mit der Säure-haltigen wässerigen Lösung und /oder einem Oxidationsmittel erreicht. Geeignete Kontaktierzeiten betragen dann mehr als 5 Minuten. In einer Ausführungsform liegen die Kontaktierzeiten im Bereich von circa 5 bis 120 Minuten, oder 5 bis 60 Minuten, oder 5 bis 20 Minuten, beispielsweise 10 Minuten.

Alternativ oder ergänzend hierzu kann die Verstärkung der Passivschicht durch wiederholtes entsprechend kurzes Kontaktieren mit der Säure-haltigen wässerigen Lösung erreicht werden. Geeignete Kontaktierzeiten betragen dann weniger als 5 Minuten, z.B. 1 - 5 Minuten, oder 2 - 4 Minuten. Idealerweise wird das kurze Kontaktieren über den Lebenszyklus des Instruments / der Oberfläche hinweg möglichst regelmäßig wiederholt, zum Beispiel im Rahmen eines standardisierten Aufbereitungsprozesses, wie dem regelmäßigen Durchlauf eines automatischen Spülprogramms in einem RDG.

In einer Ausführungsform schließt sich an Stufe (ii) Stufe (iii') an:
(iii') Entfernen der Säure-haltigen wässerigen Lösung von der Oberfläche.

In einer Ausführungsform schließt sich an Stufe (ii) oder Stufe (iii') Stufe (iii) an:
(iii) Desinfizieren der Oberfläche.

Der Begriff *"Desinfektion"* bedeutet die Reduktion, d.h. die Erniedrigung der Anzahl von Mikroorganismen, wie z.B. Keimen, Pilzen, Bakterien und / oder Viren, die sich auf der Oberfläche des Chrom-haltigen Stahls befinden oder diese besiedeln. Die Desinfektion wird in der Regel mit Wasser bei einer Temperatur von 65 - 95 °C unter Normaldruck durchgeführt. Bei der maschinellen Instrumentenaufbereitung wird die Desinfektion in der Regel im selben Gerät wie die Reinigung und im direkten Anschluss an diese durchgeführt, z.B. in einem RDG.

Typische Desinfektionsverfahren führen zu einer deutlichen Verminderung von Mikroorganismen auf den behandelten Flächen, bevorzugt zu einer Reduktion von Mikroorganismen um mindestens über 80 %, z.B. 84 - 99,95 %.

Der Begriff *"Sterilisation (Sterilisieren)"* bedeutet das Abtöten von Keimen, Pilzen, Bakterien und / oder Viren, die sich auf der Oberfläche des Chrom-haltigen Stahls befinden oder diese besiedeln. Die Sterilisation erfolgt oft durch heißen Wasserdampf unter Druck im Autoklaven, kann aber auch rein thermisch bei Temperaturen von ca. 180 - 190 °C ohne Zuführung von Wasserdampf bei Normaldruck erfolgen.

In einer weiteren Ausführungsform ist es möglich, dass das erfindungsgemäße Verfahren in einem Ultraschallbad, in einer Spülmaschine oder einem Reinigungsuns Desinfektionsgerät (RDG) durchgeführt wird.

In einer Ausführungsform wird die Spülmaschine, oder der RDG so betrieben, dass mittels eines Programms, vorzugsweise eines Computerprogramms, die Stufe (ii) aufgerufen und durchgeführt werden kann.

In einer Ausführungsform ist der Chrom-haltige Stahl Teil eines medizinischen Instruments oder ist als medizinisches Instrument ausgebildet.

Der Begriff *"medizinisches Instrument"* wird synonym zu Begriffen wie *"medizinische Vorrichtung", "medizinische Ausstattung", "zahnärztliches Instrument"* oder *"zahnärztliche Vorrichtung"* verwendet. Er schließt alle Gegenstände ein, bei denen die nicht-korrosive Entfernung von organischen oder biologischen Substanzen wie Blut, Serum, Lymphe, Proteine, Geweberückstände, Fette, Kohlenhydrate und anderen Körpersubstanzen oder Ausscheidungen eines menschlichen oder tierischen Körpers wünschenswert ist. Der Begriff schließt alle Instrumente oder Vorrichtungen ein, welche in Kontakt mit Patienten gelangen, sei es mit menschlichen oder tierischen Patienten, während der Ausübung einer medizinischen Behandlung wie einer Operation oder einer Zahnbehandlung, einer medizinischen Fußpflege oder einer Pathologie bzw. zu therapeutischen, diagnostischen und / oder Forschungszwecken. In einer Ausführungsform beinhaltet der Begriff Instrumente für die Operation, beispielsweise Skalpelle, Sonden, Klammern, Endoskope, Funktionsinstrumente für die Zahnbehandlung oder Beatmungssonden. Der Begriff umfasst auch Begriffe wie sie in einer Vielzahl von Operationsverfahren verwendet werden, sei es in einem Operationsraum eines Krankenhauses oder im Behandlungszimmer eines Arztes. Solche Instrumente werden meistens zum größten Teil aus Edelstahl hergestellt, welches die Anforderungen an medizinische Praktiken erfüllt, können jedoch zusätzlich andere Materialien enthalten. Andere Materialien sind beispielsweise Aluminium und Polypropylen oder andere Polymere. Zusätzlich umfasst der Begriff *"medizinische Instrumente"* medizinische Utensilien, welche während einer Operation oder einer anderen medizinischen Prozedur in Kontakt mit menschlichen Gewebe oder mit Blut kommen, bei der diese Utensilien verschmutzt und mikrobiologisch kontaminiert werden.

Beispiele solcher medizinischen und operativen Utensilien sind kardiovaskuläre Instrumente, Instrumente für die Augenbehandlung, mikrooperative Instrumente, neurologische und orthopädische Instrumente, Instrumente für die Laparoskopie, Endoskope, Bronchoskope, Zytoskope und Vorrichtungen für die Behandlung des Respirationstraktes; oder Instrumente, Vorrichtungen, Werkzeuge, Apparate und Ausstattungen wie sie üblicherweise in der Medizin oder Zahnmedizin verwendet werden. Diese Instrumente, Vorrichtungen und Ausrüstungen beinhalten, ohne aber darauf begrenzt zu sein: Instrumente für die Diagnostik, Tablette, Pfannen, Halter, Regale, Zangen, Scheren, Sägen (beispielsweise Knochensägen und ihre Klingen), Arterienklemmen, Messer, Meisel, Feilen, Zwickzangen, Bohrer, Raspeln, Dentalbohrer, Spreizer, Aufbrechhämmer, Hebegeräte, Klammern, Nadelhalter, Träger, Haken, Ringmesser, Aufziehvorrichtungen, Streckvorrichtungen, Extraktoren, Kellen, Spatel, Funktionskanülen, Dilatoren, Kalotten, Katheter, Stents, Artoskope, Implantate.

Beschrieben wird weiter ein Verfahren zur Aufbereitung der Oberfläche eines Chrom-haltigen Stahls, wobei die Oberfläche vorzugsweise eine Passivschicht aufweist, das dadurch gekennzeichnet ist, dass es mindestens die folgenden Stufen (i) bis (iii) aufweist, welche nacheinander ausgeführt werden:
(i) Reinigen der Oberfläche;
(ii) Kontaktieren der Oberfläche mit einer Säure-haltigen wässerigen Lösung und / oder einem Oxidationsmittel;
(iii) Desinfektion der Oberfläche.

Der Begriff *"Aufbereitung"* bedeutet typischerweise im klinischen Betrieb durchgeführte Verfahren zur Bereitstellung medizinischer Instrumente zum (ärztlichen) Gebrauch. Die Aufbereitung umfasst in der Regel Reinigung und / oder Desinfektion und / oder Sterilisation der medizinischen Instrumente. In einer bevorzugten Form umfasst der Begriff mindestens die Schritte Reinigung und Desinfektion, in einer weiteren bevorzugten Form, alle drei Schritte in der genannten Reihenfolge. In einer Ausführungsform kann der Begriff *"Reinigung"* auch den Begriff *"Verstärkung der Passivschicht"* umfassen gemäß einer oder aller der Ausführungsformen des ***ersten Aspekts*** der Erfindung.

### Stufe (i)

Zum Reinigen der Oberfläche können eine Zusammensetzung und ein Verfahren verwendet werden wie im ***ersten Aspekt*** der Erfindung definiert.

In einer Ausführungsform wird eine Enzym-Lösung eingesetzt, welche alkalisch ist, d.h. einen pH-Wert von 8 - 12 aufweist.

Demzufolge weist in einer Ausführungsform Stufe (i) auf:
(i) Reinigen der Oberfläche mit Hilfe eines Enzyms, vorzugsweise bei einem pH-Wert von 8 - 12.

Die Reinigungsstufe kann automatisch in einer Spülmaschine oder einem RDG durchgeführt werden.

### Stufe (ii)

Erfindungsgemäß erfolgt in Stufe (ii) das Kontaktieren der Oberfläche mit einer Säure-haltigen wässerigen Lösung und / oder einem Oxidationsmittel.

Der Begriff *"Kontaktieren der Oberfläche mit einer Säure-haltigen wässrigen Lösung und* / *oder einem Oxidationsmittel"* gemäß Stufe (ii) umfasst ein Verfahren, in welchem sowohl ein Teil der Oberfläche des Chrom-haltigen Stahls wie auch die vollständige Oberfläche des Chrom-haltigen Stahls mit der Lösung und / oder dem Oxidationsmittel bedeckt werden kann. Geeignete Verfahren zum Kontaktieren bestehen zum Beispiel in Sprühverfahren oder Tauchverfahren. In einer Ausführungsform wird das Kontaktieren durchgeführt unter Anwendung von Ultraschall und/oder wird mechanisch unterstützt, beispielsweise durch Bürsten.

Die Säure der Säure-haltigen wässerigen Lösung ist ausgewählt aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure. Die Verbindungen können auch in Form ihrer Salze eingesetzt werden, vorzugsweise in Form der Alkalimetallsalze oder in Form von Ammoniumsalzen.

In einer weiteren Ausführungsform kann die Säure-haltige wässerige Lösung zusätzlich zu den oben genannten Säuren oder auch getrennt davon eine oder mehrere der folgenden Säuren aufweisen: Ameisen-, Essig-, Malon-, Bernstein-, Wein-, Apfel-, Zitronen-, Glutar-, Glucon-, Milch-, Benzoe-, Ethylendiamintetraessig-, Nitrilotriessig-, Salpetersäure.

In einer weiteren Ausführungsform kann das Kontaktieren gemäß Stufe (ii) zusätzlich zum Kontaktieren mit der Säure-haltigen wässerigen lösung mit einem Oxidationsmittel erfolgen. In einer Ausführungsform ist das Oxidationsmittel ausgewählt aus einer Peroxidverbindung, vorzugsweise aus Wasserstoffperoxid oder einer Persäure wie Peressigsäure. In einer Ausführungsform liegt das Oxidationsmittel in einem Lösungsmittel vor, vorzugsweise in Wasser und / oder in einem organischen Lösungsmittel wie beim ***ersten Aspekt*** erläutert.

In einer weiteren Ausführungsform kann auch die Säure-haltige wässerige Lösung mit einem Oxidationsmittel kombiniert werden.

In einer Ausführungsform wird in Stufe (ii) das Kontaktieren so durchgeführt, dass die Passivschicht auf der Oberfläche des Stahls verstärkt wird.

Der Begriff *"Verstärkung der Passivschicht"* bedeutet in einer Ausführungsform das Erhöhen des Chrom / Eisen-Verhältnisses in der Passivschicht und / oder das Erhöhen der Schichtdicke der Passivschicht, wobei hier auf die der Dicke der Schicht Bezug genommen wird, innerhalb derer ein Chrom / Eisen-Verhältnis von mindestens ≥ 1 vorliegt. Sowohl das Chrom / Eisenverhältnis, also auch die Dicke der Passivschicht, sind mittels ESCA-Tiefenprofilmessungen bestimmbar.

Das Kontaktieren wird so durchgeführt, dass das Chrom / Eisen-Verhältnis in der Passivschicht auf einen Wert ≥ 3 erhöht wird. Eine derartige Passivschicht hat sich als wirksam gegen Korrosionsbildung erwiesen.

Das Kontaktieren wird so durchgeführt, dass die Schichtdicke der Passivschicht auf einen Wert > 4 nm erhöht wird. Eine derartige Passivschicht hat sich als wirksam gegen Korrosionsbildung erwiesen.

Das Kontaktieren wird so durchgeführt, dass das Chrom / Eisen-Verhältnis in der Passivschicht auf einen Wert ≥ 3, und die Schichtdicke der Passivschicht auf einen Wert > 4 nm erhöht wird. Eine derartige Passivschicht hat sich als besonders wirksam gegen Korrosionsbildung erwiesen.

In einer Ausführungsform wird das Kontaktieren so durchgeführt, dass das Chrom / Eisen-Verhältnis in der Passivschicht auf einen Wert ≥ 1,5 erhöht wird gemessen über eine Schichtdicke von 8 nm oder mindestens 8 nm, vorzugsweise 12 nm. Eine derartige Passivschicht hat sich als besonders wirksam gegen Korrosionsbildung erwiesen.

In einer Ausführungsform wird die Verstärkung der Passivschicht durch entsprechend langes Kontaktieren mit der Säure-haltigen wässerigen Lösung und / oder einem Oxidationsmittel erreicht. Geeignete Kontaktierzeiten betragen dann mehr als 5 Minuten. In einer Ausführungsform liegen die Kontaktierzeiten im Bereich von circa 5 bis 120 Minuten, oder 5 bis 60 Minuten, oder 5 bis 20 Minuten, beispielsweise 10 Minuten.

Alternativ oder ergänzend hierzu kann die Verstärkung der Passivschicht durch wiederholtes entsprechend kurzes Kontaktieren mit der Säure-haltigen wässerigen Lösung und / oder einem Oxidationsmittel erreicht werden. Geeignete Kontaktierzeiten betragen dann weniger als 5 Minuten, z.B. 1 - 5 Minuten, oder 2 - 4 Minuten. Idealerweise wird das kurze Kontaktieren über den Lebenszyklus des Instruments / der Oberfläche hinweg möglichst regelmäßig wiederholt, zum Beispiel im Rahmen eines standardisierten Aufbereitungsprozesses, wie dem regelmäßigen Durchlauf eines automatischen Spülprogramms in einem RDG.

In einer weiteren Ausführungsform wird die Verstärkung der Passivschicht durch entsprechende Auswahl der Säure oder der Säuren in der zum Kontaktieren verwendeten Säure-haltigen wässerigen Lösung und / oder des Oxidationsmittel erreicht. In einer Ausführungsform werden Säuren auf Basis von Maleinsäure oder Itaconsäure (Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure) verwendet wie im ***ersten Aspekt*** definiert, die sich als besonders wirksam erwiesen haben.

In einer Ausführungsform wird eine Säure-haltige wässerige Lösung verwendet, welche eine oder mehrere der folgenden Verbindungen aufweist: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure; und welche zusätzlich eine oder mehrere der folgenden Verbindungen aufweist: Ameisen-, Essig-, Malon-, Bernstein-, Wein-, Apfel-, Zitronen-, Glutar-, Glucon-, Milch-, Benzoe-, Ethylendiamintetraessig-, Nitrilotriessigsäure, vorzugsweise Zitronen- und / oder Weinsäure.

In einer weiteren Ausführungsform wird eine Säure-haltige wässerige Lösung verwendet, welche eine oder mehrere der folgenden Verbindungen aufweist: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure; und welche zusätzlich Salpetersäure aufweist.

In einer weiteren Ausführungsform wird eine Säure-haltige wässerige Lösung verwendet, welche eine oder mehrere der folgenden Verbindungen aufweist: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure; und welche zusätzlich ein Oxidationsmittel aufweist, vorzugsweise Wasserstoffperoxid oder eine Persäure wie Peressigsäure.

In einer Ausführungsform kann Stufe (ii) bei Umgebungstemperatur durchgeführt werden. Der Begriff *"Umgebungstemperatur"* bezieht sich auf Temperatur von 15 - 25 °C, z.B. circa 20 °C.

In einer weiteren Ausführungsform kann Stufe (ii) bei erhöhter Temperatur durchgeführt werden. Der Begriff *"erhöhte Temperatur"* betrifft Temperaturen oberhalb Raumtemperatur wie beispielsweise 30 bis 100 °C oder 35 bis 80 °C, oder 40 bis 70 °C, oder 50 bis 60 °C, oder 40 bis 60 °C, beispielsweise 55 °C. Die Erfinder haben gefunden, dass sich bei Durchführung der Stufe (ii) bei erhöhter Temperatur die Passivschicht sehr wirksam verstärken lässt.

In einer Ausführungsform schließt sich an Stufe (ii) Stufe (iii'):
(iii') nach Stufe (ii), Abspülen der Oberfläche mit Wasser; und vorzugsweise nach dem Abspülen, Entfernen des Wassers von der Oberfläche

Durch das Abspülen der Oberfläche wird / werden die Säure-haltige wässerige Lösung und / oder das Oxidationsmittel von der Oberfläche entfernt.

Stufe (iii') bedeutet somit: Entfernen der Säure-haltigen wässerigen Lösung und / oder des Oxidationsmittels von der Oberfläche.

Der Begriff *"Entfernen des Wassers"* bedeutet die teilweise oder vollständige Entfernung von Wasser.

### Stufe (iii)

Erfindungsgemäß wird in Stufe (iii) die Oberfläche des Chrom-haltigen Stahls desinfiziert.

Im Anschluss an die Desinfektion kann das Instrument der Sterilisation zugeführt werden.

In einer Ausführungsform wird die Oberfläche in entsalztem oder teilentsalztem Wasser erhitzt, um die Desinfektion der Chrom-haltigen Stahloberfläche zu ermöglichen. In einer Ausführungsform wird die Temperatur auf 80 - 100 °C erhöht, beispielsweise auf 90 °C. In einer Ausführungsform beträgt die Zeitdauer für die Desinfektion 1 - 15 Minuten, oder 3 - 10 Minuten, beispielsweise 5 Minuten.

In einer weiteren Ausführungsform kann die Desinfektionszeit gemäß dem A0-Wert-Konzept gemäß DIN EN ISO 15883 eingestellt werden.

In einer Ausführungsform wird das erfindungsgemäße Verfahren so durchgeführt, dass Stufe (i) die Stufen (i') und (i‴) und optional (i") aufweist; und Stufe (iii) die Stufen (iii') bis (iii‴) aufweist:
(i') Abspülen der Oberfläche mit Wasser; und vorzugsweise nach dem Abspülen, Entfernen des Wassers von der Oberfläche;
(i") optional nach Stufe (i'), Wiederholung der Stufe (i');
(i‴) nach oder während Stufe (i') oder (i"), Kontaktieren der Oberfläche mit einer Reinigungszusammensetzung; und nach dem Kontaktieren, Entfernen der Reinigungszusammensetzung von der Oberfläche;
(iii') nach Stufe (ii), Abspülen der Oberfläche mit Wasser; und vorzugsweise nach dem Abspülen, Entfernen des Wassers von der Oberfläche
(iii") Spülen der Oberfläche mit entsalztem Wasser, wobei das entsalzte Wasser erhitzt wird um die Oberfläche zu desinfizieren; und vorzugsweise nach dem Erhitzen, Entfernen des entsalzten Wassers von der Oberfläche;
(iii‴) nach Stufe (iii"), Trocknen der Oberfläche.

In einer Ausführungsform beträgt die Temperatur in Stufe (i') 5 - 30 °C. In einer Ausführungsform beträgt die Zeitdauer zur Durchführung der Stufe (i') 1 - 4 Minuten, zum Beispiel 2 - 3 Minuten.

Falls notwendig, kann der Abspülvorgang gemäß Stufe (i") wiederholt werden.

Nach dem Spülen gemäß Stufe (i') oder (i") kann eine Reinigungszusammensetzung zugesetzt werden, vorzugsweise in Form einer alkalischen Reinigungszusammensetzung. In einer Ausführungsform wird die Temperatur im Reinigungsschritt (i‴) auf eine erhöhte Temperatur wie beispielsweise 40 - 90 °C erhöht, zum Beispiel auf eine Temperatur von 50 - 60°C, beispielsweise auf eine Temperatur von 55 °C.

In einer Ausführungsform beträgt die Temperatur in Stufe (i) für einen Zeitraum von mindestens 3, oder mindestens 5, oder mindestens 10 Minuten 50 - 60 °C, zum Beispiel 55 °C.

Zum Beispiel kann die Stufe (i) bei einem pH-Wert von 10 - 12, unter Einhalten einer Temperatur von 50 - 60 °C für eine Zeitdauer von mindestens 10 Minuten durchgeführt werden. In einer Ausführungsform werden die Bereiche für pH-Wert, Temperatur, und Reinigungszeit kombiniert.

Im Anschluss an die Entfernung der Reinigungszusammensetzung wird die Oberfläche des Chrom-haltigen Stahls mit einer Säure-haltigen wässrigen Lösung und / oder einem Oxidationsmittel kontaktiert wie oben in Abschnitt *Stufe (ii)* erläutert.

Im Anschluss an das Kontaktieren gemäß Stufe (ii) wird gemäß Stufe (iii') die Oberfläche des Chrom-haltigen Stahls mit Wasser gespült, wobei vorzugsweise nach dem Spülen das Wasser von der Oberfläche entfernt wird und somit auch die Säure-haltige wässerige Lösung.

In einer Ausführungsform wird nach dem Entfernen von Wasser gemäß Stufe (iii') die Oberfläche des Chrom-haltigen Stahls mit entsalztem Wasser gespült, wobei das entsalzte Wasser erhitzt ist, um die Desinfektion der Chrom-haltigen Stahloberfläche zu ermöglichen. In einer Ausführungsform wird die Temperatur auf 80 - 100 °C erhöht, beispielsweise auf 90 °C. In einer Ausführungsform beträgt die Zeitdauer der Stufe (iii") ungefähr 1 - 10 Minuten, beispielsweise ca. 5 Minuten.

Nach der Desinfektion wird das entsalzte Wasser von der Chrom-haltigen Stahloberfläche entfernt. Dieses Wasser kann vollständig oder teilweise entfernt werden.

Nach der Entfernung des entsalzten Wassers wird die Chrom-haltige Stahloberfläche getrocknet. Zum Trocknen kann heiße Luft verwendet werden. Die Temperatur kann dabei im Bereich von 50 - 90 °C liegen.

In einer Ausführungsform ist der Chrom-haltige Stahl Teil einer im Rahmen der Instrumentenaufbereitung verwendeten Vorrichtung, wie z.B. eines Ultraschallbads, eines RDGs, oder einer Sterilisationsvorrichtung.

In einer Ausführungsform ist der Chrom-haltige Stahl Teil eines medizinischen Instruments oder ist als medizinisches Instrument ausgebildet.

In einer Ausführungsform kann das erfindungsgemäße Verfahren in einer Spülmaschine, bzw. einem RDG durchgeführt werden durchgeführt werden.

In einer Ausführungsform wird die Spülmaschine mittels eines Programms, vorzugsweise eines Computerprogramms, so betrieben, dass mittels des Programms Stufe (ii) aufgerufen und durchgeführt werden kann.

Des Weiteren wird beschrieben, dass eine Säure-haltige wässerige Lösung, wobei die Säure ausgewählt ist aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure, überraschenderweise bereits in relativ niedrigen Konzentrationen im erfindungsgemäßen Verfahren zur Verstärkung der Passivschicht verwendet werden kann.

Erfindungsgemäß beträgt der Gewichtsanteil der Verbindung oder der Verbindungen in der Lösung weniger als 5 Gew.-%, oder weniger als 3 Gew.-%, oder weniger als 1 Gew.-%, oder weniger als 0,5 Gew.-%, wie zum Beispiel 0,003 Gew.-% - 0,3 Gew.-% oder 0,01 Gew.-% - 0,1 Gew.-% bezogen auf das Gesamtgewicht der Lösung, wobei sich die Gewichts-% auf die mit der zu reinigenden Oberfläche in Kontakt zu bringenden Lösung beziehen. Der Gewichtsanteil kann demnach auch erst durch anwendungsfertige Verdünnung eines Konzentrats eingestellt werden und demnach auch erst beim automatischen Einspülen in eine Maschine, z.B. einen RDG, erreicht werden.

Ein derartiges und zur Verwendung in Schritt (ii) geeignetes Konzentrat kann auch eine Säure-haltige wässerige Lösung sein, wobei die Säure ausgewählt ist aus einer oder mehreren der folgenden Verbindungen der Gruppe A: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure; und
eine oder mehreren der folgenden Verbindungen ausgewählt aus der Gruppe B: Ameisen-, Essig-, Malon-, Bernstein-, Wein-, Apfel-, Zitronen-, Glutar-, Glucon-, Milch-, Benzoe-, Ethylendiamintetraessig-, Nitrilotriessig-, Salpetersäure;
dadurch gekennzeichnet, dass der Gewichtsanteil der Verbindung oder der Verbindungen der Gruppe A in der Lösung 10 - 20 Gew. % beträgt bezogen auf das Gesamtgewicht der Lösung; und
dass der Gewichtsanteil der Verbindung oder der Verbindungen der Gruppe B in der Lösung 10 - 20 Gew. % beträgt bezogen auf das Gesamtgewicht der Lösung; und optional kann die Summe der gesamten Gewichtsanteile der Verbindungen der Gruppen A und B in der Lösung 20 - 40 Gew.-%, bevorzugt 25 - 30 Gew.-%, betragen bezogen auf das Gesamtgewicht der Lösung.

In einer Ausführungsform besteht das Konzentrat aus den genannten Bestandteilen in den genannten Mengen, wobei die Summe der Gewichtsanteile durch Zugabe von Wasser auf insgesamt 100 Gew.-% eingestellt wird.

In einer weiteren Ausführungsform besteht das Konzentrat aus den genannten Bestandteilen in den genannten Mengen und zusätzlichen weiteren Bestandteilen von maximal 5 Gew.-%, bevorzugt maximal 3 Gew.-%, weiter bevorzugt maximal 1 Gew.-%, wobei die Summe der Gewichtsanteile durch Zugabe von Wasser auf 100 Gew.-% eingestellt wird.

In einer weiteren Ausführungsform können diese weiteren Bestandteile ausgewählt werden aus Konservierungsmitteln, oberflächenaktiven Verbindungen, Alkalien und / oder Alkoholen wie beim ***ersten Aspekt*** definiert.

In einer Ausführungsform kann in jeder dieser Ausführungsformen Maleinsäure als Verbindung A und Zitronensäure als Verbindung der Gruppe B gewählt werden.

In einer Ausführungsform liegen Maleinsäure und Zitronensäure in einem Gewichtsverhältnis von 1:1 bis 3:1, vorzugsweise 1,5:1 bis 2,5:1, weiter bevorzugt in einem Gewichtsverhältnis von 2:1 vor. In diesen Bereichen erfolgt eine besonders wirksame Passivierung.

Beschrieben wird auch eine wässerige Lösung (Säure-haltige wässerige Lösung) aufweisend eine oder mehrere der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure, die dadurch gekennzeichnet ist, dass der Gewichtsanteil der Verbindung oder der Verbindungen in der Lösung kleiner als 5 % beträgt bezogen auf das Gesamtgewicht der Lösung.

Des Weiteren wird eine Vorrichtung zur Durchführung des erfindungsgemäßen Verfahrens beschrieben.

In einer Ausführungsform ist die Vorrichtung eine Spülmaschine.

In einer Ausführungsform ist die Vorrichtung, vorzugsweise eine Spülmaschine, weiter bevorzugt eine RDG, dadurch gekennzeichnet, dass sie eine Programmierung, vorzugsweise eine Computerprogrammierung aufweist, mittels welcher mindestens die Stufe (ii) in den erfindungsgemäßen Verfahren aufgerufen und ausgeführt werden kann.

In einer Ausführungsform wird vor der ersten Anwendung oder nachdem die Oberfläche des Chrom-haltigen Stahls bereits in einer medizinischen Behandlung wie zum Beispiel einer Operation oder einer Endoskopie eingesetzt wurde, wobei am Instrument ein Peptid oder ein Protein, ein Gewebe, Fett, Fleisch, Köperflüssigkeit wie beispielsweise Blut anhaften kann, einem erfindungsgemäßen Verfahren unterzogen.

In einer Ausführungsform ist das erfindungsgemäße Verfahren ein Verfahren gemäß des ersten Aspekts oder einer der Ausführungsformen gemäß des ersten Aspekts.

Demzufolge verändert sich die Oberfläche dieses medizinischen Instruments derart, dass die Passivschicht auf der Oberfläche des Stahls verstärkt wird, durch Erhöhen des Chrom / Eisen-Verhältnisses in der
Passivschicht oder durch Erhöhen der Schichtdicke der Passivschicht oder durch Erhöhen des Chrom / Eisen-Verhältnisses in der Passivschicht und durch Erhöhen der Schichtdicke der Passivschicht.

Dem gemäß beschrieben wird ein medizinisches Instrument, welches dem oder den erfindungsgemäßen Verfahren gemäß des ersten Aspekts unterzogen wurde.

Beschrieben wird somit ein medizinisches Instrument, welches eine Oberfläche eines Chrom-haltigen Stahls aufweist, wobei der Stahl Teil des medizinischen Instruments ist oder als das medizinische Instrument ausgebildet ist, dadurch gekennzeichnet, dass es nach einem erfindungsgemäßen Verfahren herstellbar ist.

Durch die erfindungsgemäße Behandlung verändert sich das Chrom / Eisen-Verhältnis, welches vor Durchführung des erfindungsgemäßen Verfahren < 1 beträgt, auf Werte ≥ 3. Desgleichen kann die Schichtdicke der Passivschicht von einem Wert im Bereich von 1 bis 3 nm auf Werte > 4 nm erhöht werden.

Dem gemäß beschrieben wird ein medizinisches Instrument, welches eine Oberfläche eines Chrom-haltigen Stahls aufweist, wobei der Stahl Teil des medizinischen Instruments ist oder als das medizinische Instrument ausgebildet ist, und wobei die Oberfläche eine Passivschicht aufweist, dadurch gekennzeichnet, dass in der Passivschicht das Chrom / Eisen-Verhältnis ≥ 3. beträgt und / oder die Schichtdicke der Passivschicht > 4 nm beträgt.

In einer weiteren Ausführungsform ist das Chrom / Eisen-Verhältnis in der Passivschicht ≥ 1,0 oder ≥ 1,5 gemessen über eine Schichtdicke von 8 nm oder mindestens 8 nm, vorzugsweise 12 nm. Eine derartige Passivschicht hat sich als besonders wirksam gegen Korrosionsbildung erwiesen.

Weiterhin beschrieben wird ein medizinisches Instrument wie vorstehend beschrieben zur Verwendung bei der therapeutischen Behandlung eines Menschen oder eines Tieres.

Die Erfinder haben des Weiteren festgestellt, dass bei Verwendung eines Verfahrens gemäß des ersten Aspekts oder bei Verwendung einer wässrigen Lösung gemäß des zweiten Aspekts die Oberfläche des Chrom-haltigen Stahls derart verändert wird, dass das Haftungsvermögen von Prionen im Vergleich zu einer Oberfläche, welche keinem erfindungsgemäßen Verfahren unterzogen wurde, überraschenderweise herabgesetzt wird.

Prionen sind dabei Substanzen , welche für die Creutzfeldt-Jakob-Krankheit beim Menschen, für BSE (Rinderwahnsinn) beim Rind oder Scrapie beim Schaf verantwortlich gemacht werden.

Beschrieben wird weiter die Verwendung eines erfindungsgemäßen Verfahrens gemäß des ersten Aspekts oder die Verwendung eine wässerigen Lösung gemäß des zweiten Aspekts zur Herabsetzung des Haftungsvermögens von Prionen an einer Chrom-haltigen Stahloberfläche.

In einer Ausführungsform ist der Stahl Teil eines medizinischen Instruments oder ist als medizinisches Instrument ausgebildet.

Dem gemäß beschrieben wird die Verwendung der wässerigen Lösung in einem erfindungsgemäßen Verfahren gemäß des ersten Aspekts.

### BEISPIELE

### Beispiel 1

Ein fabrikneues Skalpell aus einem Werkstoff der Bezeichnung 1.4021 wurde jeweils 5-mal nacheinander für eine Zeitdauer von 10 min in einem Ultraschallbad in kochendes VE(vollentsalztes)-Wasser eingelegt. Im Anschluss wurde jeweils ein Skalpell 30 min lang in eine Zusammensetzung A (3 Gew.-% MSA in Wasser), eine Zusammensetzung B (jeweils 1 Gew.-% MSA, Weinsäure, Zitronensäure), eine Zusammensetzung C (3 Gew.-% Zitronensäure) und eine Zusammensetzung D (3 Gew.-% Weinsäure) bei einer Temperatur von 70 °C eingetaucht. Danach wurde die Zusammensetzung mit Wasser vom Skalpell abgespült und das Skalpell getrocknet. Die Zusammensetzung in der Passivschicht in Abhängigkeit von der Schichtdicke der Passivschicht wurde durch ESCA-Spektroskopie in Kombination mit Sputtern ermittelt.

Die nachfolgende Tabelle zeigt, dass die Passivschicht eines 5-mal nacheinander für eine Zeitdauer von 10 min in einem Ultraschallbad in kochendes vollentsalztes Wasser eingelegten ("unbehandelt") Skalpells durch die wässerigen Säure-haltigen Zusammensetzungen verstärkt wurde, wobei die MSA-haltigen Zusammensetzungen A und B den stärksten Effekt bezüglich der Erhöhung der Schichtdicke der Passivschicht wie auch der Erhöhung des Cr / Fe-Verhältnisses aufwiesen:

| **Sputtertiefe [nm]** | **Cr/Fe unbehandelt** | **Cr/Fe A** | **Cr/Fe B** | **Cr/Fe C** | **Cr/Fe D** |
|---|---|---|---|---|---|
| 0 | 0,3 | 4,1 | 4,2 | 4,5 | 4,30 |
| 4 | 0,8 | 4,7 | 3,5 | 2,0 | 2,0 |
| 8 | 0,5 | 2,3 | 1,7 | 1,0 | 1,0 |
| 12 | 0,6 | 2,5 | 1,5 | 1,0 | 1,0 |
| 16 | 0,7 | 2,4 | 1,5 | 1,2 | 1,2 |
| 20 | 0,8 | 2,2 | 1,4 | 1,4 | 1,4 |

### Beispiel 2

Ein Blech aus dem Werkstoff mit der Nummer 1.4021 wird in einem Ultraschallbad gemäß folgender Prozedur behandelt:
(a) Reinigung mit Mucapur AF (alkalischer Reiniger für die maschinelle Reinigung von Medizinprodukten) 1% (V/V) für 30 Minuten bei 55 °C.(b) Spülen mit VE(vollentsalztes)-Wasser
(c) Passivieren des Blechs mit der erfindungsgemäßen Lösung (Zusammensetzung: 20,0 Gew. % Maleinsäure wasserfrei, 10,0 Gew. % Zitronensäure wasserfrei); Anwendungskonzentration 1% (V/V) für 30 Minuten bei 70 °C
(d) Spülen mit VE-Wasser.

Das behandelte Blech und ein unbehandeltes Blech wurden elektrochemischen Korrosionsversuchen unterzogen, d.h. es wurde das freie Korrosionspotentials gemäß DIN 50918 gemessen. Dazu wurden die Bleche mit einem Loch versehen, so dass sie halb getaucht in eine Zelle eingehängt werden konnten. Die unbehandelte und die passivierte Probe wurden gleichzeitig gemessen, wobei das Potential kontinuierlich aufgezeichnet wurde.

Als Ergebnis wurde gefunden, dass die passivierte Probe über die gesamte Versuchszeit ein positives Potential aufwies, das um 150 mV oder höher lag als das Potential der unbehandelten Probe. Kennzeichnend war, dass relativ starke Schwankungen im Potential auftraten. Das hohe Potential signalisiert, dass die Probe im passivierten Zustand ist, d.h. dass die Passivschicht/Oxidschicht intakt ist. Die Schwankungen zeigen, dass das Potential in der Nähe des Lochkorrosionspotentials liegt und es immer wieder zur Initiierung von Korrosion kommt, die aber wieder ausheilt (repassiviert). Die Annahme, dass die Probe bei dem hohen Potential in passivierter Form vorliegt, wurde durch die Betrachtung der Proben nach dem Versuch bestätigt. Alle passivierten Proben, bei denen kein Absinken des Potentials festgestellt wurde, zeigten keine Korrosion.

Die unbehandelten Proben zeigten in ihrem Potentialverlauf ein rasches Absinken des Potentials zu negativen Werten. Dieses ist ein Kennzeichen von aktiver Korrosion. Sobald eine Korrosionsstelle ein gewisses Ausmaß erreicht hat, ist in der Regel ein Absinken zu negativen Potentialwerten festzustellen. An der Stelle, an der die Korrosion stattfindet ist keine schützende Oxidschicht mehr vorhanden.

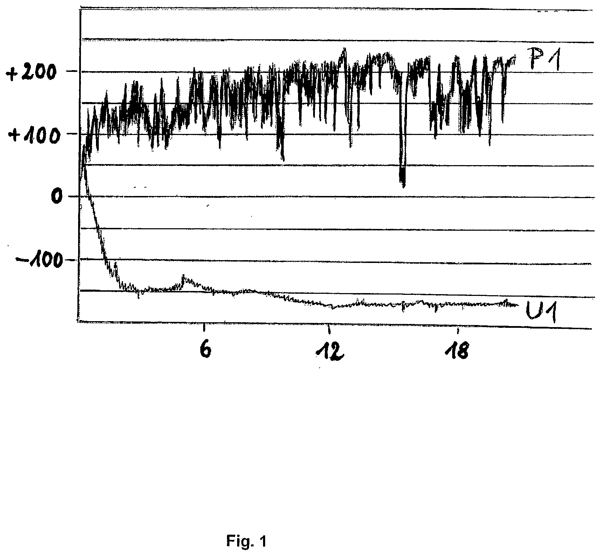

Dieses Verhalten wird in Figur 1 verdeutlicht. Auf der Ordinate ist das Potential in mV gegen eine Normalwasserstoffelektrode aufgetragen, auf der Abszisse die Zeitdauer in Stunden. Ein hohes Potential signalisiert, dass die Probe P1, welche die behandelte Probe darstellt, im passivierten Zustand ist, d.h. dass die Passivschicht intakt ist, auch nach einer Zeitdauer von ungefähr 20 Stunden. Ein rasches Absinken des Potentials zu negativen Werten ist ein Signal für aktive Korrosion. An korrodierten Teilen der Oberfläche ist keine schützende Passivschicht mehr vorhanden. Die unbehandelte Probe U1 zeigt somit bereits nach einer kurzen Zeitdauer von ca. 2 Stunden ausgeprägte Korrosion.

Durch die Versuche mit Potentialmessung wurde reproduzierbar gezeigt, dass durch die Behandlung mit dem erfindungsgemäßen Passivierungsmittel das Entstehen von sichtbarer Korrosion, die zum Aktivwerden der Probe und dadurch zum Potentialabfall führt, hinausgezögert wird.

Zusammenfassend zeigten die Versuche mit Potentialmessung, dass eine deutliche Korrosion beim Werkstoff mit der Nummer 1.4021 nach der Passivierung mit der erfindungsgemäßen Lösung später einsetzt als bei unbehandelten Proben. Das Passivierungsmitteln hat also einen positiven Effekt auf das Korrosionsverhalten des Werkstoffs.

## Patentansprüche

1. Verfahren zur Verstärkung der Passivschicht auf der Oberfläche eines Chrom-haltigen Stahls durch Erhöhen des Chrom / Eisen-Verhältnisses in der Passivschicht und / oder durch Erhöhen der Schichtdicke der Passivschicht, **dadurch gekennzeichnet, dass** es mindestens die Stufe (ii) aufweist und optional vor oder gleichzeitig mit Stufe (ii) die Stufe (i) ausgeführt wird:
(i) Reinigen der Oberfläche;
(ii) Kontaktieren der Oberfläche des Stahls mit einer Säure-haltigen wässerigen Lösung, wobei die Säure ausgewählt ist aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure, wobei das wasserlösliches Copolymerisat von Maleinsäure oder Maleinsäureanhydrid oder Itaconsäure ein Copolymerisat von Maleinsäure oder Maleinsäureanhydrid oder Itaconsäure mit ethylenisch ungesättigten Verbindungen oder mit anderen ungesättigten Verbindungen ist;
wobei der Gewichtsanteil von Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure in der Säure-haltigen wässerigen Lösung kleiner als 5 Gew.-% bezogen auf das Gesamtgewicht der Lösung ist,
wobei das Kontaktieren der Oberfläche mit der Säure-haltigen wässrigen Lösung so durchgeführt wird, dass das Chrom / Eisen-Verhältnis in der Passivschicht auf einen Wert ≥ 3 und die Schichtdicke der Passivschicht auf einen Wert > 4 nm erhöht wird.

2. Verfahren nach Anspruch 1, wobei die Säure-haltige wässerige Lösung zusätzlich eine oder mehrere der folgenden Verbindungen oder Zusammensetzungen aufweist:
Ameisen-, Essig-, Malon-, Bernstein-, Wein-, Apfel-, Zitronen-, Glutar-, Glucon-, Milch-, Benzoe-, Ethylendiamintetraessig-, Nitrilotriessig-, Salpetersäure;
Oxidationsmittel; Reinigungszusammensetzung.

3. Verfahren nach Anspruch 1 oder 2, wobei besagter Gewichtsanteil durch Verdünnung eines Konzentrats eingestellt wird, wobei das zur Verwendung in Schritt (ii) geeignete Konzentrat eine Säure-haltige wässerige Lösung ist, wobei die Säure ausgewählt ist aus einer oder mehreren der folgenden Verbindungen der Gruppe A: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure; und eine oder mehreren der folgenden Verbindungen ausgewählt aus der Gruppe B:
Ameisen-, Essig-, Malon-, Bernstein-, Wein-, Apfel-, Zitronen-, Glutar-, Glucon-, Milch-, Benzoe-, Ethylendiamintetraessig-, Nitrilotriessig-, Salpetersäure;
wobei der Gewichtsanteil der Verbindung oder der Verbindungen der Gruppe A in der Lösung 10 - 20 Gew. % beträgt bezogen auf das Gesamtgewicht der Lösung; und
wobei der Gewichtsanteil der Verbindung oder der Verbindungen der Gruppe B in der Lösung 10 - 20 Gew. % beträgt bezogen auf das Gesamtgewicht der Lösung; und
optional die Summe der gesamten Gewichtsanteile der Verbindungen der Gruppen A und B in der Lösung 20 - 40 Gew.-%, bevorzugt 25 - 30 Gew.-%, beträgt bezogen auf das Gesamtgewicht der Lösung.

4. Verfahren nach einem der vorstehenden Ansprüche, wobei die Säure-haltige wässerige Lösung Maleinsäure umfasst.

5. Verfahren nach einem der vorstehenden Ansprüche, wobei die Säure-haltige wässerige Lösung Maleinsäure und Zitronensäure umfasst.

6. Verfahren nach Anspruch 5, wobei Maleinsäure und Zitronensäure in einem Gewichtsverhältnis von 1:1 bis 3:1, vorzugsweise 1,5:1 bis 2,5:1, insbesondere 2:1 vorliegen.

7. Verfahren nach einem oder mehreren der Ansprüche 4-6, wobei der Gewichtsanteil von Maleinsäure in der Säure-haltigen wässerigen Lösung bezogen auf das Gesamtgewicht der Lösung kleiner als 4 Gew.-% ist.

8. Verfahren nach einem der vorstehenden Ansprüche, wobei die Säure-haltige wässerige Lösung vorliegt als Flüssigkeit; oder als Schaum; oder als Gel; oder als Paste; oder als Spray; und / oder in einem Vlies; in einem Ultraschallbad; in einer Spülmaschine oder in einem Reinigungs- und Desinfektionsgerät.

9. Verfahren nach einem der vorstehenden Ansprüche, wobei sich an Stufe (ii) Stufe (iii') anschließt:
(iii') Entfernen der Säure-haltigen wässerigen Lösung von der Oberfläche.

10. Verfahren nach einem der vorstehenden Ansprüche, wobei sich an Stufe (ii) oder Stufe (iii') die Stufe (iii) anschließt:
(iii) Desinfizieren der Oberfläche.

11. Verfahren nach Anspruch 1, wobei die Oberfläche eines Chrom-haltigen Stahls aufbereitet wird, wobei der Begriff "Aufbereitung" im klinischen Bereich durchgeführte Verfahren zur Bereitstellung medizinischer Instrumente zum (ärztlichen) Gebrauch bedeutet, **dadurch gekennzeichnet, dass** es mindestens die folgenden Stufen (i) bis (iii) aufweist, welche nacheinander ausgeführt werden:
(i) Reinigen der Oberfläche;
(ii) Kontaktieren der Oberfläche mit einer Säure-haltigen wässerigen Lösung, wobei die Oberfläche optional ferner mit einem Oxidationsmittel kontaktiert wird;
(iii) Desinfizieren der Oberfläche.

12. Verfahren nach Anspruch 11, wobei Stufe (i) die Stufen (i') und (i‴) und optional (i") aufweist; und Stufe (iii) die Stufen (iii') bis (iii‴) aufweist:
(i') Abspülen der Oberfläche mit Wasser; und vorzugsweise nach dem Abspülen, Entfernen des Wassers von der Oberfläche;
(i") optional nach Stufe (i'), Wiederholung der Stufe (i');
(i‴) nach oder während Stufe (i') oder (i"), Kontaktieren der Oberfläche mit einer Reinigungszusammensetzung; und nach dem Kontaktieren, Entfernen der Reinigungszusammensetzung von der Oberfläche;
(iii') nach Stufe (ii), Abspülen der Oberfläche mit Wasser; und vorzugsweise nach dem Abspülen, Entfernen des Wassers von der Oberfläche
(iii") Spülen der Oberfläche mit entsalztem Wasser, wobei das entsalzte Wasser erhitzt wird, um die Oberfläche zu desinfizieren; und vorzugsweise nach dem Erhitzen, Entfernen des entsalzten Wassers von der Oberfläche;
(iii‴) nach Stufe (iii"), Trocknen der Oberfläche.

13. Verfahren nach einem der Ansprüche 11 oder 12, wobei die Säure-haltige wässerige Lösung zusätzlich eine oder mehrere der folgenden Verbindungen aufweist: Ameisen-, Essig-, Malon-, Bernstein-, Wein-, Apfel-, Zitronen-, Glutar-, Glucon-, Milch-, Benzoe-, Ethylendiamintetraessig-, Nitrilotriessig-, Salpetersäure.

14. Verfahren nach einem der vorstehenden Ansprüche, wobei der Chrom-haltige Stahl ausgewählt ist aus einem Stahl mit der Bezeichnung 1.4301, 1.4404, 1.4021 oder 1.4024.

15. Verfahren nach einem der vorstehenden Ansprüche, wobei der Stahl Teil eines medizinischen Instruments ist oder als medizinisches Instrument ausgebildet ist.

16. Verfahren nach einem der vorstehenden Ansprüche, wobei das Verfahren in einer Spülmaschine oder einem Reinigungs- und Desinfektionsgerät (RDG) durchgeführt wird.

17. Verwendung einer Säure-haltigen wässrigen Lösung zur Verstärkung der Passivschicht auf der Oberfläche eines Chrom-haltigen Stahls durch Erhöhen des Chrom / Eisen-Verhältnisses in der Passivschicht und / oder durch Erhöhen der Schichtdicke der Passivschicht,
wobei die Säure ausgewählt ist aus einer oder mehreren der folgenden Verbindungen: Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure, wobei das wasserlösliches Copolymerisat von Maleinsäure oder Maleinsäureanhydrid oder Itaconsäure ein Copolymerisat von Maleinsäure oder Maleinsäureanhydrid oder Itaconsäure mit ethylenisch ungesättigten Verbindungen oder mit anderen ungesättigten Verbindungen ist;
wobei der Gewichtsanteil von Maleinsäure, Maleinsäureanhydrid, Maleinsäurehalbester, Itaconsäure, Itaconsäurehalbester, wasserlösliches Copolymerisat von Maleinsäure oder Maleinsäureanhydrid oder Itaconsäure in der Säure-haltigen wässerigen Lösung kleiner als 5 Gew.-% bezogen auf das Gesamtgewicht der Lösung ist,

## Claims

1. Process for strengthening the passive layer on the surface of a chromium-containing steel by increasing the chromium/iron ratio in the passive layer and/or by increasing the layer thickness of the passive layer, **characterized in that** it comprises at least step (ii) and optionally step (i) is carried out before or simultaneously with step (ii):
(i) cleaning the surface;
(ii) contacting the surface of the steel with an acid-containing aqueous solution, wherein the acid is selected from one or more of the following compounds: maleic acid, maleic anhydride, maleic half-ester, itaconic acid, itaconic half-ester, water-soluble copolymer of maleic acid or maleic anhydride or itaconic acid, wherein the water-soluble copolymer of maleic acid or maleic anhydride or itaconic acid is a copolymer of maleic acid or maleic anhydride or itaconic acid with ethylenically unsaturated compounds or with other unsaturated compounds;
wherein the proportion by weight of maleic acid, maleic anhydride, maleic half-ester, itaconic acid, itaconic half-ester, water-soluble copolymer of maleic acid or maleic anhydride or itaconic acid in the acid-containing aqueous solution is less than 5% by weight based on the total weight of the solution,
wherein the contacting of the surface with the acid-containing aqueous solution is carried out such that the chromium/iron ratio in the passive layer is increased to a value ≥ 3 and the layer thickness of the passive layer is increased to a value > 4 nm.

2. Process according to claim 1, wherein the acid-containing aqueous solution additionally comprises one or more of the following compounds or compositions: formic, acetic, malonic, succinic, tartaric, malic, citric, glutaric, gluconic, lactic, benzoic, ethylenediaminetetraacetic, nitrilotriacetic, nitric acid; oxidizing agent; cleaning composition.

3. Process according to claim 1 or 2, wherein said proportion by weight is adjusted by diluting a concentrate, wherein the concentrate suitable for use in step (ii) is an acid-containing aqueous solution, wherein the acid is selected from one or more of the following compounds of group A: maleic acid, maleic anhydride, maleic half-ester, itaconic acid, itaconic half-ester, water-soluble copolymer of maleic acid or maleic anhydride or itaconic acid; and one or more of the following compounds selected from group B:
formic, acetic, malonic, succinic, tartaric, malic, citric, glutaric, gluconic, lactic, benzoic, ethylenediaminetetraacetic, nitrilotriacetic, nitric acid;
wherein the proportion by weight of the compound or compounds of group A in the solution is 10-20% by weight based on the total weight of the solution; and
wherein the proportion by weight of the compound or compounds of group B in the solution is 10-20% by weight based on the total weight of the solution; and
optionally, the sum of the total proportions by weight of the compounds of groups A and B in the solution is 20 - 40% by weight, preferably 25 - 30% by weight, based on the total weight of the solution.

4. Process according to any one of the preceding claims, wherein the acid-containing aqueous solution comprises maleic acid.

5. Process according to any one of the preceding claims, wherein the acid-containing aqueous solution comprises maleic acid and citric acid.

6. Process according to claim 5, wherein maleic acid and citric acid are present in a weight ratio of 1:1 to 3:1, preferably 1.5:1 to 2.5:1, in particular 2:1.

7. Process according to one or more of claims 4-6, wherein the proportion by weight of maleic acid in the acid-containing aqueous solution is less than 4% by weight based on the total weight of the solution.

8. Process according to any one of the preceding claims, wherein the acid-containing aqueous solution is present as a liquid; or as a foam; or as a gel; or as a paste; or as a spray; and/or in a fleece; in an ultrasonic bath; in a dishwasher; or in a washer-disinfector.

9. Process according to any one of the preceding claims, wherein step (ii) is followed by step (iii'):
(iii') removing of the acid-containing aqueous solution from the surface.

10. Process according to any one of the preceding claims, wherein step (ii) or step (iii') is followed by step (iii):
(iii) disinfecting the surface.

11. Process according to claim 1, wherein the surface of a chromium-containing steel is reprocessed, wherein the term "reprocessing" means processes carried out in the clinical field for providing medical instruments for (medical) use, **characterized in that** it comprises at least the following steps (i) to (iii), which are carried out in succession:
(i) cleaning the surface;
(ii) contacting the surface with an acid-containing aqueous solution, optionally further contacting the surface with an oxidizing agent;
(iii) disinfecting the surface.

12. Process of claim 11, wherein step (i) comprises steps (i') and (i‴) and optionally (i"); and step (iii) comprises steps (iii') to (iii‴):
(i') rinsing the surface with water; and preferably after rinsing, removing the water from the surface;
(iʺ) optionally after step (i'), repetition of step (i');
(i‴) after or during step (i') or (i"), contacting the surface with a cleaning composition; and after the contacting, removing the cleaning composition from the surface;
(iii') after step (ii), rinsing the surface with water; and preferably after rinsing, removing the water from the surface
(iii") rinsing the surface with desalinated water, wherein the desalinated water is heated, to disinfect the surface; and preferably after heating, removing the desalinated water from the surface;
(iii‴) after step (iii"), drying the surface.

13. Process of any one of claims 11 or 12, wherein the acid-containing aqueous solution additionally comprises one or more of the following compounds: formic, acetic, malonic, succinic, tartaric, malic, citric, glutaric, gluconic, lactic, benzoic, ethylenediaminetetraacetic, nitrilotriacetic, nitric acid.

14. Process according to any one of the preceding claims, wherein the chromium-containing steel is selected from a steel designated 1.4301, 1.4404, 1.4021 or 1.4024.

15. Process according to any one of the preceding claims, wherein the steel is part of a medical instrument or is designed as a medical instrument.

16. Process according to any one of the preceding claims, wherein the method is carried out in a dishwasher or a washer-disinfector (WD).

17. Use of an acid-containing aqueous solution to strengthen the passive layer on the surface of a chromium-containing steel by increasing the chromium/iron ratio in the passive layer and/or by increasing the layer thickness of the passive layer,
wherein the acid is selected from one or more of the following compounds: maleic acid, maleic anhydride, maleic half-ester, itaconic acid, itaconic half-ester, water-soluble copolymer of maleic acid or maleic anhydride or itaconic acid, wherein the water-soluble copolymer of maleic acid or maleic anhydride or itaconic acid is a copolymer of maleic acid or maleic anhydride or itaconic acid with ethylenically unsaturated compounds or with other unsaturated compounds;
wherein the proportion by weight of maleic acid, maleic anhydride, maleic half-ester, itaconic acid, itaconic half-ester, water-soluble copolymer of maleic acid or maleic anhydride or itaconic acid in the acid-containing aqueous solution is less than 5% by weight based on the total weight of the solution.

## Revendications

1. Procédé, destiné à renforcer la couche passive sur la surface d'un acier contenant du chrome par augmentation du rapport chrome / fer dans la couche passive et/ou par augmentation de l'épaisseur de couche de la couche passive, **caractérisé en ce qu'**il comporte au moins l'étape (ii) et, **en ce qu'**en option, l'étape (i) est réalisée antérieurement ou simultanément à l'étape (ii) :
(i) nettoyage de la surface ;
(ii) mise en contact de la surface de l'acier avec une solution aqueuse acide, l'acide étant sélectionné parmi un ou plusieurs des composés suivants : l'acide maléique, l'anhydride maléique, un semi-ester de l'acide maléique, l'acide itaconique, un semi-ester de l'acide itaconique, un copolymère hydrosoluble de l'acide maléique ou de l'anhydride maléique ou de l'acide itaconique, le copolymère hydrosoluble de l'acide maléique ou de l'anhydride maléique ou de l'acide itaconique étant un copolymère de l'acide maléique ou de l'anhydride maléique ou de l'acide itaconique avec des composés éthyléniquement insaturés ou avec d'autres composés insaturés ;
la part en poids de l'acide maléique, de l'anhydride maléique, du semi-ester de l'acide maléique, de l'acide itaconique, du semi-ester de l'acide itaconique, du copolymère hydrosoluble de l'acide maléique ou de l'anhydride maléique ou de l'acide itaconique dans la solution aqueuse acide étant inférieure à 5 % en poids, rapportés au poids total de la solution,
la mise en contact de la surface avec la solution aqueuse acide étant réalisée de telle sorte que le rapport chrome / fer dans la couche passive soit augmenté à une valeur ≥ 3 et que l'épaisseur de couche de la couche passive soit augmentée à une valeur > 4 nm.

2. Procédé selon la revendication 1, la solution aqueuse acide comportant additionnellement un ou plusieurs des composés suivants : l'acide formique, l'acide acétique, l'acide malonique, l'acide succinique, l'acide tartrique, l'acide malique, l'acide citrique, l'acide glutarique, l'acide gluconique, l'acide lactique, l'acide benzoïque, l'acide éthylène-diamine-tétraacétique, l'acide nitrilotriacétique, l'acide nitrique ; un produit oxydant ; une composition nettoyante.

3. Procédé selon la revendication 1 ou 2, la part en poids citée étant réglée par dilution d'un concentré, le concentré apte à être utilisé dans l'étape (ii) étant une solution aqueuse acide, l'acide étant sélectionné parmi un ou plusieurs des composés suivants du groupe A : l'acide maléique, l'anhydride maléique, un semi-ester de l'acide maléique, l'acide itaconique, un semi-ester de l'acide itaconique, un copolymère hydrosoluble de l'acide maléique ou de l'anhydride maléique ou de l'acide itaconique; et un ou plusieurs des composés, suivants, sélectionnés dans le groupe B :
l'acide formique, l'acide acétique, l'acide malonique, l'acide succinique, l'acide tartrique, l'acide malique, l'acide citrique, l'acide glutarique, l'acide gluconique, l'acide lactique, l'acide benzoïque, l'acide éthylène-diamine-tétraacétique, l'acide nitrilotriacétique, l'acide nitrique ;
la part en poids du composé ou des composés du groupe A dans la solution s'élevant à de 10 à 20 % en poids, rapportés au poids total de la solution ; et la part en poids du composé ou des composés du groupe B dans la solution s'élevant à de 10 à 20 % en poids, rapportés au poids total de la solution ; et en option, la somme des parts en poids totales des composés des groupes A et B dans la solution s'élevant à de 20 à 40 % en poids, de préférence à de 25 à 30 % en poids, rapportés au poids total de la solution.

4. Procédé selon l'une quelconque des revendications précédentes, la solution aqueuse acide comprenant de l'acide maléique.

5. Procédé selon l'une quelconque des revendications précédentes, la solution aqueuse acide comprenant de l'acide maléique et de l'acide citrique.

6. Procédé selon la revendication 5, l'acide maléique et l'acide citrique étant présents selon un rapport en poids de 1 : 1 à 3 : 1, de préférence de 1,5 : 1 à 2,5 : 1, notamment de 2 : 1.

7. Procédé selon l'une quelconque ou plusieurs des revendications 4 à 6, la part en poids de l'acide maléique dans la solution aqueuse acide étant inférieure à 4 % en poids, rapportés au poids total de la solution.

8. Procédé selon l'une quelconque des revendications précédentes, la solution aqueuse acide se présentant sous la forme d'un liquide ; ou d'une mousse ; ou d'un gel; ou d'une pâte ; ou d'un spray ; et/ou dans un non-tissé ; dans un bain ultrasonique ; dans une machine à laver ou dans un appareil de nettoyage et de désinfection.

9. Procédé selon l'une quelconque des revendications précédentes, à l'étape (ii) faisant suite l'étape (iii') :
(iii') retrait de la solution aqueuse acide de la surface.

10. Procédé selon l'une quelconque des revendications précédentes, à l'étape (ii) ou à l'étape (iii') faisant suite l'étape (iii) :
(iii) désinfection de la surface.

11. Procédé selon la revendication 1, la surface d'un acier contenant du chrome étant traitée, le terme de « traitement » se traduisant par un procédé réalisé dans le domaine clinique pour mettre à disposition des instruments médicaux pour leur utilisation (médicale), **caractérisé en ce qu'**il comporte au moins les étapes (i) à (iii) suivantes, lesquelles sont réalisées successivement :
(i) nettoyage de la surface ;
(ii) mise en contact de la surface avec une solution aqueuse acide, en option, la surface étant mise en contact par ailleurs avec un produit oxydant ;
(iii) désinfection de la surface.

12. Procédé selon la revendication 11, l'étape (i) comportant les étapes (i′) et (i‴) et en option, l'étape (iʺ) ; et l'étape (iii) comportant les étapes (iii') à (iii‴) :
(i′) rinçage de la surface à l'eau ; et de préférence après le rinçage, retrait de l'eau de la surface ;
(iʺ) en option après l'étape (i'), réitération de l'étape (i′) ;
(i‴) après ou pendant l'étape (i′) ou (i"), mise en contact de la surface avec une composition nettoyante ; et après la mise en contact, retrait de la composition nettoyante de la surface ;
(iii′) après l'étape (ii), rinçage de la surface à l'eau ; et de préférence après le rinçage, retrait de l'eau de la surface ;
(iii") rinçage de la surface à l'eau déminéralisée, l'eau déminéralisée étant chauffée, pour désinfecter la surface ; et de préférence, après l'avoir chauffée, retrait de l'eau déminéralisée de la surface ;
(iii‴) après l'étape (iii"), séchage de la surface.

13. Procédé selon l'une quelconque des revendications 11 ou 12, la solution aqueuse acide comportant additionnellement un ou plusieurs des composés suivants : l'acide formique, l'acide acétique, l'acide malonique, l'acide succinique, l'acide tartrique, l'acide malique, l'acide citrique, l'acide glutarique, l'acide gluconique, l'acide lactique, l'acide benzoïque, l'acide éthylène-diamine-tétraacétique, l'acide nitrilotriacétique, l'acide nitrique.

14. Procédé selon l'une quelconque des revendications précédentes, l'acier contenant du chrome étant sélectionné parmi un acier portant la désignation 1.4301, 1.4404, 1.4021 ou 1.4024.

15. Procédé selon l'une quelconque des revendications précédentes, l'acier étant une partie d'un instrument médical ou étant conçu sous la forme d'un instrument médical.

16. Procédé selon l'une quelconque des revendications précédentes, le procédé étant réalisé dans une machine à laver ou dans un appareil de nettoyage et de désinfection (LD).

17. Utilisation d'une solution aqueuse acide pour renforcer la couche passive sur la surface d'un acier contenant du chrome par augmentation du rapport chrome / fer dans la couche passive et/ou par augmentation de l'épaisseur de couche de la couche passive,
l'acide étant sélectionné parmi un ou plusieurs des composés suivants : l'acide maléique, l'anhydride maléique, un semi-ester de l'acide maléique, l'acide itaconique, un semi-ester de l'acide itaconique, un copolymère hydrosoluble de l'acide maléique ou de l'anhydride maléique ou de l'acide itaconique, le copolymère hydrosoluble de l'acide maléique ou de l'anhydride maléique ou de l'acide itaconique étant un copolymère de l'acide maléique ou de l'anhydride maléique ou de l'acide itaconique avec des composants éthyléniquement insaturés ou avec d'autres composés insaturés ;
la part en poids de l'acide maléique, de l'anhydride maléique, du semi-ester de l'acide maléique, de l'acide itaconique, du semi-ester de l'acide itaconique du copolymère hydrosoluble de l'acide maléique ou de l'anhydride maléique ou de l'acide itaconique dans la solution aqueuse acide étant inférieure à 5 % en poids, rapportés au poids total de la solution.
